# EUROPEAN PATENT SPECIFICATION

(11) **EP 2 113 567 B1**
(45) Date of publication and mention of the grant of the patent: **03.04.2019**
(21) Application number: 09165187.7
(22) Date of filing: 21.12.2007
(51) Int. Cl.: C12N 15/11, A61K 48/00, C12Q 1/68

(54) **MicroRNA target site blocking oligos and uses thereof**
MicroRNA Zielstellen-blockierende Oligonucleotide und deren Verwendung
Oligonucléotides bloqueurs de site cibles de microRNA et leur utilisation

(30) Priority: 21.12.2006 DK 200601695
(43) Date of publication of application: 04.11.2009
(62) Divisional of application: 07846435.1
(73) Proprietor: QIAGEN GmbH, 40724 Hilden (DE)
(72) Inventor: Echwald, Søren Morgenthaler, 3050 Humlebaek (DK); Lund, Anders Henrik, 2500 Valby (DK); Frankel, Lisa, 2100 Copenhagen Ø (DK); Rasmussen, Søren Vestergaard, 2990 Nivaa (DK); Aristarkhov, Alexander, Boston, MA 02467 (US)
(74) Representative: Aera A/S

(56) References cited:
- WO-A-2005/111211
- WO-A-2006/069584
- CN-A- 101 054 576
- CN-A- 101 054 580
- US-A1- 2007 065 840
- WANG XIU-JIE ET AL: "Prediction and identification of Arabidopsis thaliana microRNAs and their mRNA targets" GENOME BIOLOGY, BIOMED CENTRAL LTD., LONDON, GB, vol. 5, no. 9, 31 August 2004 (2004-08-31), page R65, XP021012924 ISSN: 1465-6906
- JOHN BINO ET AL: "Human MicroRNA targets." PLOS BIOLOGY NOV 2004, vol. 2, no. 11, November 2004 (2004-11), page e363, XP002481762 ISSN: 1545-7885
- VATOLIN ET AL: "A Novel Method to Detect Functional MicroRNA Targets" JOURNAL OF MOLECULAR BIOLOGY, LONDON, GB, vol. 358, no. 4, 12 May 2006 (2006-05-12), pages 983-996, XP005392551 ISSN: 0022-2836
- LEWIS B P: "Prediction of Mammalian MicroRNA Targets" CELL, CELL PRESS, CAMBRIDGE, NA, US, vol. 115, no. 7, 26 December 2003 (2003-12-26), pages 787-798, XP002295226 ISSN: 0092-8674
- LIM L P ET AL: "Microarray analysis shows that some microRNAs downregulate large numbers of target mRNAs" NATURE, NATURE PUBLISHING GROUP, LONDON, vol. 433, no. 7027, 17 February 2005 (2005-02-17), pages 769-773, XP002994329 ISSN: 0028-0836
- XIAO JIENING ET AL: "Novel approaches for gene-specific interference via manipulating actions of microRNAs: examination on the pacemaker channel genes HCN2 and HCN4." JOURNAL OF CELLULAR PHYSIOLOGY AUG 2007, vol. 212, no. 2, August 2007 (2007-08), pages 285-292, XP002481763 ISSN: 0021-9541
- Jiening Xiao ET AL: "Retracted: Novel approaches for gene-specific interference via manipulating actions of microRNAs: Examination on the pacemaker channel genes HCN2 and HCN4", Journal of Cellular Physiology, vol. 212, no. 2, 21 August 2007 (2007-08-21), pages 285-292, XP055199875, ISSN: 0021-9541, DOI: 10.1002/jcp.21062

## Description

The present invention relates to nucleic acids designed to prevent the binding of endogenous or exogenous microRNA and uses thereof.

### Background of the Invention

The present invention relates to the study and modulation of the effect of small RNAs on target nucleotide sequences in a wide variety of nucleic acid samples and more specifically to the methods employing the design and use of oligonucleotides that are useful for preventing the binding of endogenous or exogenous microRNA especially to RNA target sequences, such as microRNA target sites.

### MicroRNAs

The expanding inventory of international sequence databases and the concomitant sequencing of nearly 200 genomes representing all three domains of life - bacteria, archea, and eukaryota - have been the primary drivers in the process of deconstructing living organisms into comprehensive molecular catalogs of genes, transcripts, and proteins. The importance of the genetic variation within a single species has become apparent, extending beyond the completion of genetic blueprints of several important genomes, culminating in the publication of the working draft of the human genome sequence in 2001 (Lander, Linton, Birren et al., 2001 Nature 409: 860-921; Venter, Adams, Myers et al., 2001 Science 291: 1304-1351; Sachidanandam, Weissman, Schmidt et al., 2001 Nature 409: 928-933). On the other hand, the increasing number of detailed, large-scale molecular analyses of transcription originating from the human and mouse genomes along with the recent identification of several types of non-protein-coding RNAs, such as small nucleolar RNAs, siRNAs, microRNAs and antisense RNAs, indicate that the transcriptomes of higher eukaryotes are much more complex than originally anticipated (Wong et al. 2001, Genome Research 11: 1975-1977; Kampa et al. 2004, Genome Research 14: 331-342).

As a result of the Central Dogma: 'DNA makes RNA, and RNA makes protein', RNAs have been considered as simple molecules that just translate the genetic information into protein. Recently, it has been estimated that although most of the genome is transcribed, almost 97% of the genome does not encode proteins in higher eukaryotes, but putative, non-coding RNAs (Wong et al. 2001, Genome Research 11: 1975-1977). The non-coding RNAs (ncRNAs) appear to be particularly well suited for regulatory roles that require highly specific nucleic acid recognition. Therefore, the view of RNA is rapidly changing from the merely informational molecule to comprise a wide variety of structural, informational and catalytic molecules in the cell.

Recently, a large number of small non-coding RNA genes have been identified and designated as microRNAs (miRNAs) (for review, see Ke et al. 2003, Curr.Opin. Chem. Biol. 7:516-523). The first miRNAs to be discovered were the lin-4 and let-7 that are heterochronic switching genes essential for the normal temporal control of diverse developmental events (Lee et al. 1993, Cell 75:843-854; Reinhart et al. 2000, Nature 403: 901-906) in the roundworm *C. elegans.* miRNAs have been evolutionarily conserved over a wide range of species and exhibit diversity in expression profiles, suggesting that they occupy a wide variety of regulatory functions and exert significant effects on cell growth and development (Ke et al. 2003, Curr. Opin. Chem. Biol. 7:516-523). Recent work has shown that miRNAs can regulate gene expression at many levels, representing a novel gene regulatory mechanism and supporting the idea that RNA is capable of performing similar regulatory roles as proteins. Understanding this RNA-based regulation will help us to understand the complexity of the genome in higher eukaryotes as well as understand the complex gene regulatory networks.

miRNAs are 19-25 nucleotide (nt) RNAs that are processed from longer endogenous hairpin transcripts (Ambros et al. 2003, RNA 9: 277-279). To date more than 1345 microRNAs have been identified in humans, worms, fruit flies and plants according to the miRNA registry database release 5.0 in September 2004, hosted by Sanger Institute, UK, and many miRNAs that correspond to putative genes have also been identified. Some miRNAs have multiple loci in the genome (Reinhart et al. 2002, Genes Dev. 16: 1616-1626) and occasionally, several miRNA genes are arranged in tandem clusters (Lagos-Quintana et al. 2001, Science 294: 853-858). The fact that many of the miRNAs reported to date have been isolated just once suggests that many new miRNAs will be discovered in the future. A recent in-depth transcriptional analysis of the human chromosomes 21 and 22 found that 49 % of the observed transcription was outside of any known annotation, and furthermore, that these novel transcripts were both coding and non-coding RNAs (Kampa et al. 2004, Genome Research 14: 331-342). The identified miRNAs to date represent most likely the tip of the iceberg, and the number of miRNAs might turn out to be very large.

The combined characteristics of microRNAs characterized to date (Ke et al. 2003, Curr.Opin. Chem. Biol. 7:516-523; Lee et al. 1993, Cell 75:843-854; Reinhart et al. 2000, Nature 403: 901-906) can be summarized as:
1. miRNAs are single-stranded RNAs of about 19-25 nt that regulate the expression, stability, and /or translation into protein of complementary messenger RNAs
2. They are cleaved from a longer endogenous double-stranded hairpin precursor by the enzyme Dicer.
3. miRNAs match precisely the genomic regions that can potentially encode precursor RNAs in the form of double-stranded hairpins.
4. miRNAs and their predicted precursor secondary structures are phylogenetically conserved.

Several lines of evidence suggest that the enzymes Dicer and Argonaute are crucial participants in miRNA biosynthesis, maturation, and function (Grishok et al. 2001, Cell 106: 23-24). Mutations in genes required for miRNA biosynthesis lead to genetic developmental defects, which are, at least in part, derived from the role of generating miRNAs. The current view is that miRNAs are cleaved by Dicer from the hairpin precursor in the form of duplex, initially with 2 or 3 nt overhangs in the 3' ends, and are termed pre-miRNAs. Cofactors join the pre-miRNP and unwind the pre-miRNAs into single-stranded miRNAs, and pre-miRNP is then transformed to miRNP. miRNAs can recognize regulatory targets while part of the miRNP complex. There are several similarities between miRNP and the RNA-induced silencing complex, RISC, including similar sizes and both containing RNA helicase and the PPD proteins. It has therefore been proposed that miRNP and RISC are the same RNP with multiple functions (Ke et al. 2003, Curr.Opin. Chem. Biol. 7:516-523). Different effectors direct miRNAs into diverse pathways. The structure of pre-miRNAs is consistent with the observation that 22 nt RNA duplexes with 2 or 3 nt overhangs at the 3' ends are beneficial for reconstitution of the protein complex and might be required for high affinity binding of the short RNA duplex to the protein components (for review, see Ke et al. 2003, Curr.Opin. Chem. Biol. 7:516-523).

Growing evidence suggests that miRNAs play crucial roles in eukaryotic gene regulation. The first miRNA genes to be discovered, lin-4 and let-7, base-pair incompletely to repeated elements in the 3' untranslated regions (UTRs) of other heterochronic genes, and regulate the translation directly and negatively by antisense RNA-RNA interaction (Lee et al. 1993, Cell 75:843-854; Reinhart et al. 2000, Nature 403: 901-906). Other miRNAs are thought to interact with target mRNAs by limited complementary and suppressed translation as well (Lagos-Quintana et al. 2001, Science 294: 853-858; Lee and Ambros 2001, Science 294: 858-862). Many studies have shown, however, that given a perfect complementarity between miRNAs and their target RNA, could lead to target RNA degradation rather than inhibit translation (Hutvagner and Zamore 2002, Science 297: 2056-2060), suggesting that the degree of complementarity determines their functions. By identifying sequences with near complementarity, several targets have been predicted, most of which appear to be potential transcriptional factors that are crucial in cell growth and development. The high percentage of predicted miRNA targets acting as developmental regulators and the conservation of target sites suggest that miRNAs are involved in a wide range of organism development and behaviour and cell fate decisions (for review, see Ke et al. 2003, Curr.Opin. Chem. Biol. 7:516-523). For example, John et al. 2004 (PLoS Biology 2: e363) used known mammalian miRNAs to scan the 3' untranslated regions (UTRs) from human, mouse and rat genomes for potential miRNA target sites using a scanning algorithm based on sequence complementarity between the mature miRNA and the target site, binding energy of the miRNA:mRNA duplex and evolutionary conservation. They identified a total of 2307 target mRNAs conserved across the mammals with more than one target site at 90 % conservation of target site sequence and 660 target genes at 100 % conservation level. Scanning of the two fish genomes; *Danio rerio* (zebrafish) and *Fugu rubripes* (Fugu) identified 1000 target genes with two or more conserved miRNA sites between the two fish species (John et al. 2004 PLoS Biology 2: e363). Among the predicted targets, particularly interesting groups included mRNA encoding transcription factors, components of the miRNA machinery, other proteins involved in the translational regulation as well as components of the ubiquitin machinery. Wang et al. 2004 (Genome Biology 5:R65) have developed and applied a computational algorithm to predict 95 Arabidopsis thaliana miRNAs, which included 12 known ones and 83 new miRNAs. The 83 new miRNAs were found to be conserved with more than 90 % sequence identity between the Arabidopsis and rice genomes. Using the Smith-Waterman nucleotide-alignment algorithm to predict mRNA targets for the 83 new miRNAs and by focusing on target sites that were conserved in both Arabidopsis and rice, Wang et al. 2004 (Genome Biology 5:R65) predicted 371 mRNA targets with an average of 4.8 targets per miRNA. A large proportion of these mRNA targets encoded proteins with transcription regulatory activity.

### MicroRNAs and human disease

Analysis of the genomic location of miRNAs indicates that they play important roles in human development and disease. Several human diseases have already been pinpointed in which miRNAs or their processing machinery might be implicated. One of them is spinal muscular atrophy (SMA), a paediatric neurodegenerative disease caused by reduced protein levels or loss-of-function mutations of the survival of motor neurons (SMN) gene (Paushkin et al. 2002, Curr.Opin.Cell Biol. 14: 305-312). Two proteins (Gemin3 and Gemin4) that are part of the SMN complex are also components of miRNPs, whereas it remains to be seen whether miRNA biogenesis or function is dysregulated in SMA and what effect this has on pathogenesis. Another neurological disease linked to mi/siRNAs is fragile X mental retardation (FXMR) caused by absence or mutations of the fragile X mental retardation protein (FMRP)(Nelson et al. 2003, TIBS 28: 534-540), and there are additional clues that miRNAs might play a role in other neurological diseases. Yet another interesting finding is that the miR-224 gene locus lies within the minimal candidate region of two different neurological diseases: early-onset Parkinsonism and X-linked mental retardation (Dostie et al. 2003, RNA: 9: 180-186). Links between cancer and miRNAs have also been recently described. The most frequent single genetic abnormality in chronic lymphocytic leukaemia (CLL) is a deletion localized to chromosome 13q14 (50% of the cases). A recent study determined that two different miRNA (miR15 and miR16) genes are clustered and located within the intron of LEU2, which lies within the deleted minimal region of the B-cell chronic lymphocytic leukaemia (B-CLL) tumour suppressor locus, and both genes are deleted or down-regulated in the majority of CLL cases (Calin et al. 2002, Proc. Natl. Acad. Sci.U.S.A. 99: 15524-15529). It has been anticipated that connections between miRNAs and human diseases will only strengthen in parallel with the knowledge of miRNAs and the gene networks that they control. Moreover, the understanding of the regulation of RNA-mediated gene expression is leading to the development of novel therapeutic approaches that will be likely to revolutionize the practice of medicine (Nelson et al. 2003, TIBS 28: 534-540).

### Detection and analysis of microRNAs

The current view that miRNAs may represent a newly discovered, hidden layer of gene regulation has resulted in high interest among researchers around the world in the discovery of miRNAs, their targets and mechanism of action. Detection and analysis of these small RNAs is, however not trivial. Thus, the discovery of more than 1400 miRNAs to date has required taking advantage of their special features. First, the research groups have used the small size of the miRNAs as a primary criterion for isolation and detection. Consequently, standard cDNA libraries would lack miRNAs, primarily because RNAs that small are normally excluded by size selection in the cDNA library construction procedure. Total RNA from fly embryos, worms or HeLa cells have been size fractionated so that only molecules 25 nucleotides or smaller would be captured (Moss 2002, Curr.Biology 12: R138-R140). Synthetic oligomers have then been ligated directly to the RNA pools using T4 RNA ligase. Then the sequences have been reverse-transcribed, amplified by PCR, cloned and sequenced (Moss 2002, Curr.Biology 12: R138-R140). The genome databases have subsequently been queried with the sequences, confirming the origin of the miRNAs from these organisms as well as placing the miRNA genes physically in the context of other genes in the genome. The vast majority of the cloned sequences have been located in intronic regions or between genes, occasionally in clusters, suggesting that the tandemly arranged miRNAs are processed from a single transcript to allow coordinated regulation. Furthermore, the genomic sequences have revealed the fold-back structures of the miRNA precursors (Moss 2002, Curr.Biology 12: R138-R140).

The size and often low level of expression of different miRNAs require the use of sensitive and quantitative analysis tools. Due to their small size of 19-25 nt, the use of quantitative real-time PCR for monitoring expression of mature miRNAs is excluded. Therefore, most miRNA researchers currently use Northern blot analysis combined with polyacrylamide gels to examine expression of both the mature and pre-miRNAs (Reinhart et al. 2000, Nature 403: 901-906; Lagos-Quintana et al. 2001, Science 294: 853-858; Lee and Ambros 2001, Science 294: 862-864). Primer extension has also been used to detect the mature miRNA (Zeng and Cullen 2003, RNA 9: 112-123). The disadvantage of all the gel-based assays (Northern blotting, primer extension, RNase protection assays etc.) as tools for monitoring miRNA expression includes low throughput and poor sensitivity. Consequently, a large amount of total RNA per sample is required for Northern analysis of miRNAs, which is not feasible when the cell or tissue source is limited.

DNA microarrays would appear to be a good alternative to Northern blot analysis to quantify miRNAs in a genome-wide scale, since microarrays have excellent throughput. Krichevsky et al. 2003 used cDNA microarrays to monitor the expression of miRNAs during neuronal development with 5 to 10 µg aliquot of input total RNA as target, but the mature miRNAs had to be separated from the miRNA precursors using micro concentrators prior to microarray hybridizations (Krichevsky et al. 2003, RNA 9: 1274-1281). Liu et al 2004 (Liu et al. 2004, Proc.Natl. Acad. Sci, U.S.A 101:9740-9744) have developed a microarray for expression profiling of 245 human and mouse miRNAs using 40-mer DNA oligonucleotide capture probes. Thomson *et al.* 2004 (Thomson et al. 2004, Nature Methods 1: 1-6) describe the development of a custom oligonucleotide microarray platform for expression profiling of 124 mammalian miRNAs conserved in human and mouse using oligonucleotide capture probes complementary to the mature microRNAs. The microarray was used in expression profiling of the 124 miRNAs in question in different adult mouse tissues and embryonic stages. A similar approach was used by Miska et al. 2004 (Genome Biology 2004; 5:R68) for the development of an oligoarray for expression profiling of 138 mammalian miRNAs, including 68 miRNAs from rat and monkey brains. Yet another approach was taken by Barad et al. 2004 (Genome Research 2004; 14: 2486-2494), who developed a 60-mer oligonucleotide microarray platform for known human mature miRNAs and their precursors. The drawback of all DNA-based oligonucleotide arrays regardless of the capture probe length is the requirement of high concentrations of labelled input target RNA for efficient hybridization and signal generation, low sensitivity for rare and low-abundant miRNAs, and the necessity for post-array validation using more sensitive assays such as real-time quantitative PCR, which is not currently feasible. In addition, at least in some array platforms discrimination of highly homologous miRNA differing by just one or two nucleotides could not be achieved, thus presenting problems in data interpretation, although the 60-mer microarray by Barad et al. 2004 (Genome Research 2004; 14: 2486-2494) appears to have adequate specificity.

A PCR approach has also been used to determine the expression levels of mature miRNAs (Grad et al. 2003, Mol. Cell 11: 1253-1263). This method is useful to clone miRNAs, but highly impractical for routine miRNA expression profiling, since it involves gel isolation of small RNAs and ligation to linker oligonucleotides. Allawi et al. (2004, RNA 10: 1153-1161) have developed a method for quantification of mature miRNAs using a modified Invader assay. Although apparently sensitive and specific for the mature miRNA, the drawback of the Invader quantification assay is the number of oligonucleotide probes and individual reaction steps needed for the complete assay, which increases the risk of cross-contamination between different assays and samples, especially when high-throughput analyses are desired. Schmittgen et al. (2004, Nucleic Acids Res. 32: e43) describe an alternative method to Northern blot analysis, in which they use real-time PCR assays to quantify the expression of miRNA precursors. The disadvantage of this method is that it only allows quantification of the precursor miRNAs, which does not necessarily reflect the expression levels of mature miRNAs. In order to fully characterize the expression of large numbers of miRNAs, it is necessary to quantify the mature miRNAs, such as those expressed in human disease, where alterations in miRNA biogenesis produce levels of mature miRNAs that are very different from those of the precursor miRNA. For example, the precursors of 26 miRNAs were equally expressed in non-cancerous and cancerous colorectal tissues from patients, whereas the expression of mature human miR143 and miR145 was greatly reduced in cancer tissues compared with non-cancer tissues, suggesting altered processing for specific miRNAs in human disease (Michael et al. 2003, Mol. Cancer Res. 1: 882-891). On the other hand, recent findings in maize with miR166 and miR165 in *Arabidopsis thaliana,* indicate that microRNAs act as signals to specify leaf polarity in plants and may even form movable signals that emanate from a signalling centre below the incipient leaf (Juarez et al. 2004, Nature 428: 84-88; Kidner and Martienssen 2004, Nature 428: 81-84).

Most of the miRNA expression studies in animals and plants have utilized Northern blot analysis, tissue-specific small RNA cloning, and expression profiling by microarrays or real-time PCR of the miRNA hairpin precursors, as described above. However, these techniques lack the resolution for addressing the spatial and temporal expression patterns of mature miRNAs. Due to the small size of mature miRNAs, detection of them by standard RNA *in situ* hybridization has proven difficult to adapt in both plants and vertebrates, even though *in situ* hybridization has recently been reported in *A. thaliana* and maize using RNA probes corresponding to the stem-loop precursor miRNAs (Chen et al. 2004, Science 203: 2022-2025; Juarez et al. 2004, Nature 428: 84-88). Brennecke et al. 2003 (Cell 113: 25-36) and Mansfield et al. 2004 (Nature Genetics 36: 1079-83) report on an alternative method in which reporter transgenes, so-called sensors, are designed and generated to detect the presence of a given miRNA in an embryo. Each sensor contains a constitutively expressed reporter gene (e.g. lacZ or green fluorescent protein) harbouring miRNA target sites in its 3'-UTR. Thus, in cells that lack the miRNA in question, the transgene RNA is stable allowing detection of the reporter, whereas cells expressing the miRNA, the sensor mRNA is targeted for degradation by the RNAi pathway. Although sensitive, this approach is time-consuming since it requires generation of the expression constructs and transgenes. Furthermore, the sensor-based technique detects the spatiotemporal miRNA expression patterns via an indirect method as opposed to direct *in situ* hybridization of the mature miRNAs.

The large number of miRNAs along with their small size makes it difficult to create loss-of-function mutants for functional genomics analyses. Another potential problem is that many miRNA genes are present in several copies per genome occurring in different loci, which makes it even more difficult to obtain mutant phenotypes. Boutla et al. 2003 (Nucleic Acids Research 31: 4973-4980) describe the use of DNA antisense oligonucleotides complementary to 11 different miRNAs in *Drosophila* as well as their use to inactivate the miRNAs by injecting the DNA oligonucleotides into fly emryos. Of the 11 DNA antisense oligonucleotides, only 4 constructs showed severe interference with normal development, while the remaining 7 oligonucleotides didn't show any phenotypes presumably due to their inability to inhibit the miRNA in question. Thus, the succes rate for using DNA antisense oligonucleotides to inhibit miRNA function would most likely be too low to allow functional analyses of miRNAs on a larger, genomic scale. An alternative approach to this has been reported by Hutvagner et al. 2004 (PLoS Biology 2: 1-11), in which 2'-O-methyl antisense oligonucleotides could be used as potent and irreversible inhibitors of miRNA function *in vitro* and *in vivo* in *Drosophila* and *C. elegans,* thereby inducing a loss-of-function phenotype. A drawback of this method is the need of high 2'-O-methyl oligonucleotide concentrations (100 micromolar) in transfection and injection experiments, which may be toxic to the animal.

In conclusion, a challenge in functional analysis and therapeutic modulation of the mature miRNAs using currently available methods is the ability of microRNAs to intereact with target nucleic acids through imperfect target site recognition and hence for each microRNA to target multiple target nucleotides in an undesired manner. Current methods for inhibition of miRNA activity such as the anti-miRNA oligonucleotides (Weiler et al, Gene Ther 13; 496-502, 2005), the 'antagomirs' (Krutzfeldt et al., Nature 438; 685-689, 2005) and microRNA 'sponges' (Ebert et al., Nature Methods, 4(9); 721-726, 2007) inhibit the activity of the targeted microRNA by a reduction in miRNA/target interaction over a broad range.

WO2008/061537 discloses a method for producing oligonucleotides binding to miRNAs binding sites that spans the entire miRNA binding site and an adjacent region of a transcript.

WO2006/069584 discloses miRNA binding sequences comprising nucleic acid analogs (LNA) and their use for the detection of miRNAs.

The present invention provides the design and development of novel oligonucleotide compositions and sequences, providing an accurate, specific, and highly sensitive solution to specifically block a particular microRNA target site in a particular target nucleic acid without inducing degradation of the same target nucleic acid.

### Summary of the Invention

The challenges of establishing genome function and understanding the layers of information hidden in the complex transcriptomes of higher eukaryotes call for novel, improved technologies for detection and analysis of non-coding RNA and protein-coding RNA molecules in complex nucleic acid samples. Thus, it would be highly desirable to be able to detect and analyse the expression of mature microRNAs using methods based on specific and sensitive oligonucleotide detection probes.

The present invention solves the current problems faced by conventional approaches used in studying and modulating the interaction of mature miRNAs with their target nucleic acid(s) (e.g., mRNAs) by providing a method for the design, synthesis, and use of novel oligonucleotide compositions with improved sensitivity and high sequence specificity for RNA target sequences. Such oligonucleotides include a recognition sequence at least partially complementary to the microRNA target sequence, wherein the recognition sequence is substituted with high-affinity nucleotide analogues, e.g., LNA, to increase the sensitivity and specificity relative to conventional oligonucleotides, such as DNA oligonucleotides, for hybridization to short target sequences, e.g., mature miRNAs and stem-loop precursor miRNAs.

In one preferred aspect, the present invention relates to a method for producing a microRNA target site blocking nucleic acid directed at a specific transcript, wherein an oligonucleotide sequence of the nucleic acid overlaps the 5' or 3' end of the microRNA target site, and at the same time partly overlaps the non-microRNA target sites, the method comprising the steps of:
1) selecting a known microRNA having at least one identified or putative microRNA target site,
2) obtaining the sequence of said microRNA and the sequence of one of said microRNA target sites,
3) selecting and obtaining the sequence of a transcript which comprises the microRNA target site selected in step 2,
4) designing a microRNA target site blocking nucleic acid which is at least 10 nucleotides long and comprises at least one LNA analogue and which is either
   a) at least partially complementary to the microRNA target site and which comprises a consecutive sequence of nucleotides which are complementary to the 3' end of the microRNA target site selected in step 3 and a consecutive sequence of nucleotides which are complementary to the transcript sequence immediately adjacent and downstream of the 3' end of the microRNA target site, or
   b) at least partially complementary to the microRNA target site and which comprises a consecutive sequence of nucleotides which are complementary to end 5' end of the microRNA target site selected in step 3 and a consecutive sequence of nucleotides which are complementary to the transcript sequence immediately adjacent and upstream of the 5' end of the microRNA target site
5) synthesizing the nucleic acid designed in step 4.

Accordingly, in one aspect, the invention provides a nucleic acid including a high affinity nucleic acid analog, e.g., LNA, and binding to a region including a portion of an miRNA target site and a naturally occurring nucleic acid sequence adjacent to the miRNA target site. The nucleic acid binds, for example, to the 3' end or 5' end of the miRNA target site. Alternatively, the nucleic acid binds to 100% of the miRNA target site. In another embodiment, at least 10%, e.g., at least 15, 20, 25, 30, 35, 40, 45, 50, 55, 60, 65, 70, 75, 80, 85, 90, or 95%, of the nucleic acid is not complementary to the miRNA target site. The nucleic acid is, for example, from 5-30 nucleotides, e.g., at least 10, 15, 20, or 25. In certain embodiments, the nucleic acid includes a plurality of high affinity nucleotide analogs, e.g., of the same or different type. For example, the nucleic acid may include up to 80%, e.g., up to 75, 70, 65, 60, 55, 50, 45, 40, 35, 30, 25, or 20 %, of the high affinity nucleic acid analog or the high affinity nucleic acid analog, e.g., LNA, in combination with one or more additional analogs, e.g., 2' OMe. The plurality of analogs may be disposed so that no more than four naturally occurring nucleotides occur in linear sequence.

The nucleic acid is typically complementary to at least two nucleotides of the miRNA target site and at least three nucleotides in the naturally occurring nucleic acid sequence adjacent to the miRNA target site. The nucleic acid may be complementary to 3-8 nucleotides of the miRNA target site to which the seed sequence of the miRNA binds. For certain nucleic acids, the miRNA targeted binds to more than one target in a genome, and the naturally occurring nucleic acid sequence adjacent to the miRNA target site differs by three or more nucleotides from other such sequences.

A high affinity nucleic analog may or may not be disposed at the 3' or 5' end of the nucleic acid. The nucleic acid is also preferably RNase resistant. Preferably, the nucleic acid does not prevent production of the miRNA from its corresponding pri- or pre-miRNA. In other embodiments, the analogs are not disposed in regions capable of forming auto-dimers or intramolecular complexes.

The binding of the nucleic acid to the region desirably reduces the binding of the miRNA to the region, e.g., by at least 50%, 55%, 60%, 65%, 70%, 75%, 80%, 85%, or 90%. Alternatively, the nucleic acid binds to the region with a lower Kd than the miRNA in vivo. The nucleic acid may also have an increase in binding affinity to the region as determined by an increase in Tm of at least 2°C, compared to the naturally occurring RNA complement of the region.

Preferred miRNAs are those associated with cancer, heart disease, cardiovascular disease, neurological diseases such as Parkinson's disease, Alzheimer's, spinal muscular atrophy and X mental retardation, atherosclerosis, postangioplasty restenosis, transplantation arteriopathy, stroke, viral infection, psoriasis, metabolic disease, diabetes mellitus, and diabetic nephropathy.

In various embodiments, a nucleic acid of the invention specifically includes one or more of 2'-O-methyl-modified nucleic acids (2'-OMe), 2'-O-(2-methoxyethyl)-modified nucleic acids (2'-MOE), 2'-Deoxy-2'-fluoro-β-D-arabinoic acid (FANA), Cyclohexene nucleic acids (CeNA), Hexitol nucleic acids (HNA) and analogs thereof, Intercalating Nucleic Acids (INA), 2'-O,4'-C-Ethylene-bridged-Nucleic Acids (ENA), and peptide nucleic acid (PNA). In other embodiments, a nucleic acid of the invention does not include 2'-O-methyl-modified nucleic acids (2'-OMe); a nucleic acid of the invention does not include 2'-O-(2-methoxyethyl)-modified nucleic acids (2'-MOE); a nucleic acid of the invention does not include 2'-Deoxy-2'-fluoro-β-D-arabinoic acid (FANA); a nucleic acid of the invention does not include Cyclohexene nucleic acids (CeNA); a nucleic acid of the invention does not include Hexitol nucleic acids (HNA) or analogs thereof; a nucleic acid of the invention does not include Intercalating Nucleic Acids (INA); a nucleic acid of the invention does not include 2'-O,4'-C-Ethylene-bridged-Nucleic Acids (ENA); and/or a nucleic acid of the invention does not include peptide nucleic acids (PNA).

The invention also features a pharmaceutical composition including one or more nucleic acids of the invention and a pharmaceutically acceptable excipient. Pharmaceutical compositions may be used in treatment of diseases associate with miRNA, as described herein. The invention also includes a diagnostic kit including one or more nucleic acids of the invention. The diagnostic kits may be employed to diagnose a disease associated with an miRNA, to prognose a subject having a disease associated with an miRNA, to determine the risk of a subject to develop a disease associated with an miRNA, or to determine the efficacy of a particular treatment for a disease associated with an miRNA. The nucleic acids of the invention may further be used as research tools and in drug screening, as described herein.

The invention further features a method of inhibiting the binding of an miRNA to a target site by contacting one or more nucleic acids of the invention with a cell expressing the target site. The contacting may occur in vitro, e.g., in drug screening, or in vivo, e.g., in therapy.

In another aspect, the invention features a method of identifying the presence of an miRNA target site by contacting a nucleic acid sample from a subject with one or more nucleic acids of the invention and determining whether the one or more nucleic acid binds to the sample. Such methods may be employed diagnostically, as described.

In a further aspect, the invention features a method of verifying the presence of a miRNA target site by contacting a nucleic acid sample from a subject with one or more nucleic acids of the invention and determining an expression level of a nucleic acid comprising said target site or its translation product, wherein a change in the expression level of the nucleic acid comprising the target site or its translation product verifies the presence of the miRNA target site.

Furthermore, the invention features a method of verifying the presence of a miRNA target site, said method comprising predicting the presence of a miRNA target site in a nucleic acid, such as by using a target site prediction algorithm, and contacting the nucleic acid sample with one or more nucleic acids of the invention and determining an expression level of a nucleic acid comprising the target site or its translation product, wherein a change in the expression level of a nucleic acid comprising the target site or its translation product verifies the presence of the miRNA target site.

The invention also features a method of treating a disease caused by binding of an miRNA to a target site by contacting a subject with one or more nucleic acids of the invention in an amount sufficient to reduce binding of the miRNA at the target site, e.g., by at least 10%, 15%, 20%, 25%, 30%, 35%, 40%, 45%, 50%, 55%, 60%, 65%, 70%, 75%, 80%, 85%, 90%, or 95%. Exemplary diseases associated with miRNA are provided herein.

The nucleic acids of the invention are not splice-splice switching oligomers, e.g., of the TNFR superfamily (U.S. 2007/0105807).

In another aspect, the invention provides a nucleic acid as described above with the expection that the region does not include a naturally occurring nucleic acid sequence adjacent to the miRNA target site. Such nucleic acids may be used in any of the methods described herein and have any of the features of the other nucleic acids of the invention, unless otherwise noted.

### Brief Description of the Drawings

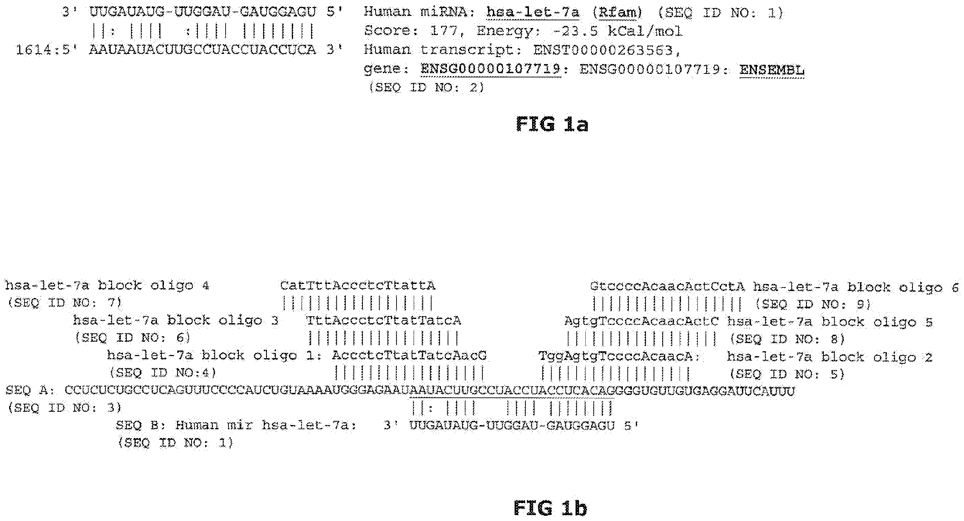

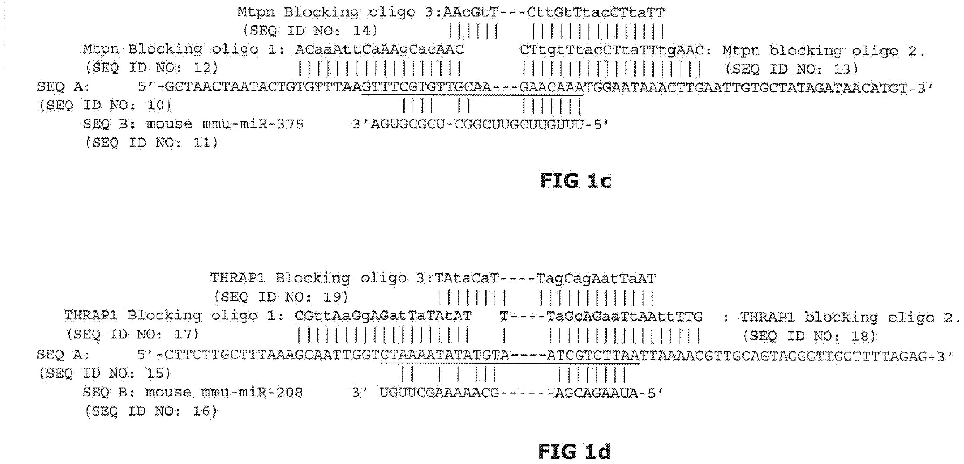

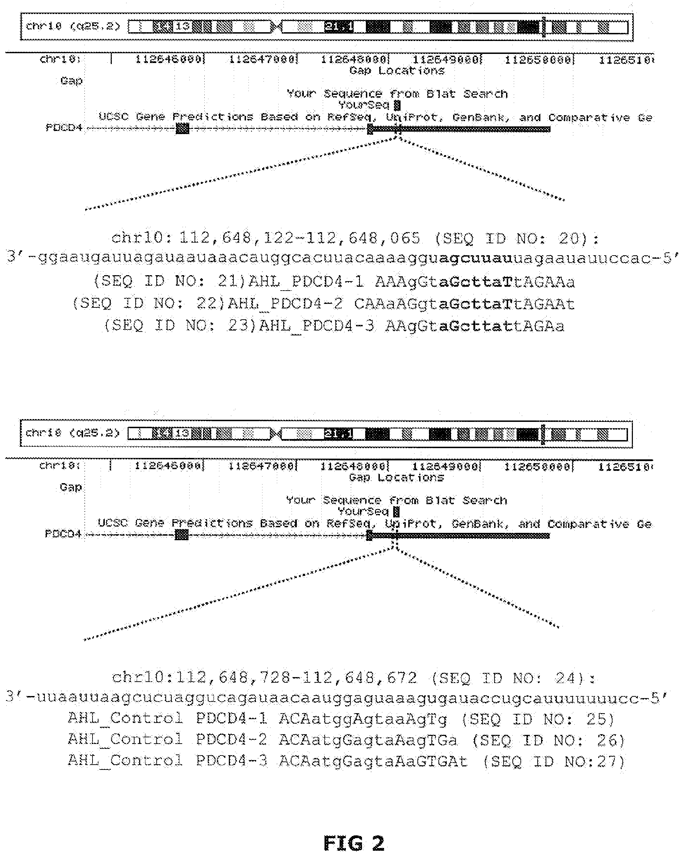

Figure 1: Examples of blocking oligos for a microRNA target site.
   (a) Human mir hsa-let-7a sequence and alignment to human transcript ENST00000263563.
   (b) Exemplary microRNA target site blocking oligos for human microRNA hsa-let-7a target site starting at position 1614 in human transcript: ENST00000263563, gene: ENSG00000107719. SEQ A: partial sequence of target gene ENSG00000107719. Underlined sequence; region of mir target site. SEQ B: Sequence of Human mir hsa-let-7a and alignment to partial sequence of target gene ENSG00000107719. hsa-let-7a block oligo 1-6: sequences of suggested blocking oligos for blocking target sites for hsa-let-7a on target gene ENSG00000107719. LNA moieties are marked with capital letters.
      Predicted Tms of blocking oligos:
      hsa-let-7a block oligo 1: Tm= 59
      hsa-let-7a block oligo 2: Tm= 71
      hsa-let-7a block oligo 3: Tm= 52
      hsa-let-7a block oligo 4: Tm= 54
      hsa-let-7a block oligo 5: Tm= 54
      hsa-let-7a block oligo 6: Tm= 67
   (c) Exemplary microRNA target site blocking oligos for mouse microRNA mmu-miR-375, in the Mtpn gene Refseq: NM_008098.3. SEQ A: partial sequence of target gene Mtpn. Underlined sequence; region of mir target site. SEQ B: Mouse mmu-miR-375 and alignment to partial sequence of target gene Mtpn. Mtpn blocking oligos 1-3: sequences of suggested blocking oligos for blocking target sites for mmu-miR-375 on target gene Mtpn. LNA moieties are marked with capital letters.
      Predicted Tms of blocking oligos:
      Mtpn blocking oligo 1: Tm= 71
      Mtpn blocking oligo 2: Tm= 66
      Mtpn blocking oligo 3: Tm= 68
   (d) Exemplary microRNA target site blocking oligos for mouse microRNA mmu-miR-208 in the 3'UTR of Med13/THRAP1 gene RefSeq: NM_001080931.1. SEQ A: partial sequence of target gene Med13/THRAP1. Underlined sequence; region of mir target site. SEQ B: Mouse mmu-miR-208 and alignment to partial sequence of target gene Med13/THRAP1. THRAP1 blocking oligos 1-3: sequences of suggested blocking oligos for blocking target sites for mmu-miR-208 on target gene Med13/THRAP1. LNA moieties are marked with capital letters.
      Predicted Tms of blocking oligos:
      THRAP1 blocking oligo 1: Tm= 68
      THRAP1 blocking oligo 2: Tm= 69
      THRAP1 blocking oligo 3: Tm= 64
Figure 2: Blocking oligos for the miR-21 target site in the PDCD4 gene transcript. Localization and partial sequences of the 3'UTR of the PDCD4 gene transcript. Sequences of three blocking oligos, AHL-PDCD4-1, AHL-PDCD4-2 and AHL-PDCD4-3, recognizing the miR-21 target site in the 3'UTR of PDCD4 gene transcript and three control oligos, AHL_Control PDCD4-1, AHL_Control PDCD4-2 and AHL_Control PDCD4-3, recognizing a different part of the 3'UTR of PDCD4 gene transcript. LNA moieties are marked with capital letters. The seed sequence of miR-21 is marked with bold.

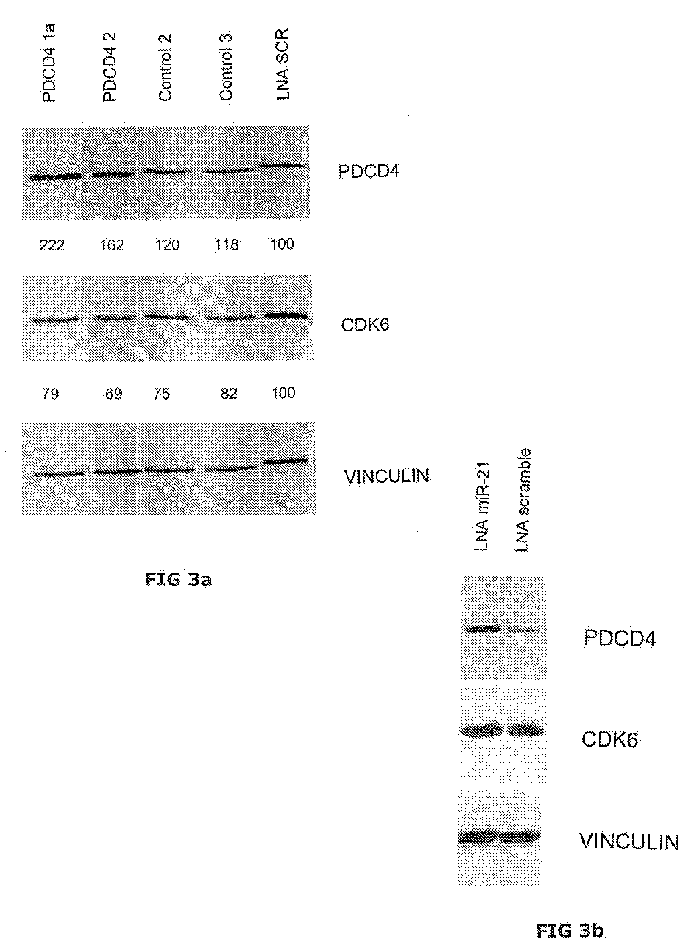

Figure 3: Western blot of cell extracts from MCF-7 cells transfected with blocking oligos and control oligos. Cells were transfected with 50 nM of the indicated LNA constructs. Western blot was performed using antibodies against PDCD4, CDK6, and Vinculin. Bands were quantified relative to the appropriate Vinculin loading controls, and the relative quantifications are shown. PDCD4 1a, AHL_PDCD4-1. PDCD4 2, AHL_PDCD4-2. Control 2, AHL_Control PDCD4-2. Control 3, AHL_Control PDCD4-3. LNA SCR, LNA scramble (Exiqon, NB-1 negative, lot nr. EQ29093).

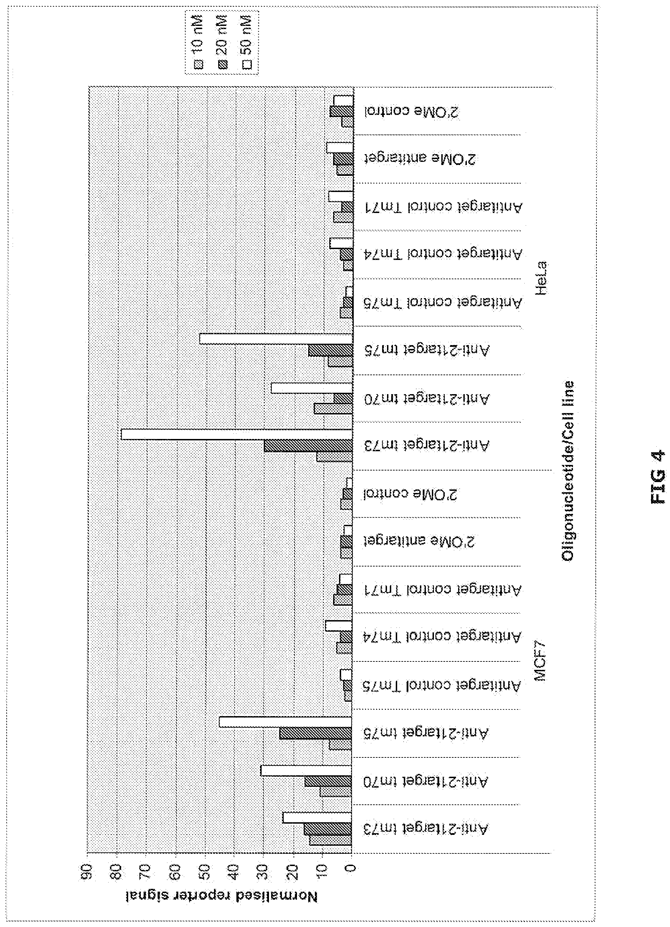

Figure 4: Target site blocking pMIR-21 luciferase vector. The diagram shows the normalised expression level of the pMIR-21 reporter vector as a function of oligonucleotide concentration. The left panel shows results obtained from HeLa cells and the right panel results obtained from MCF7 cells.

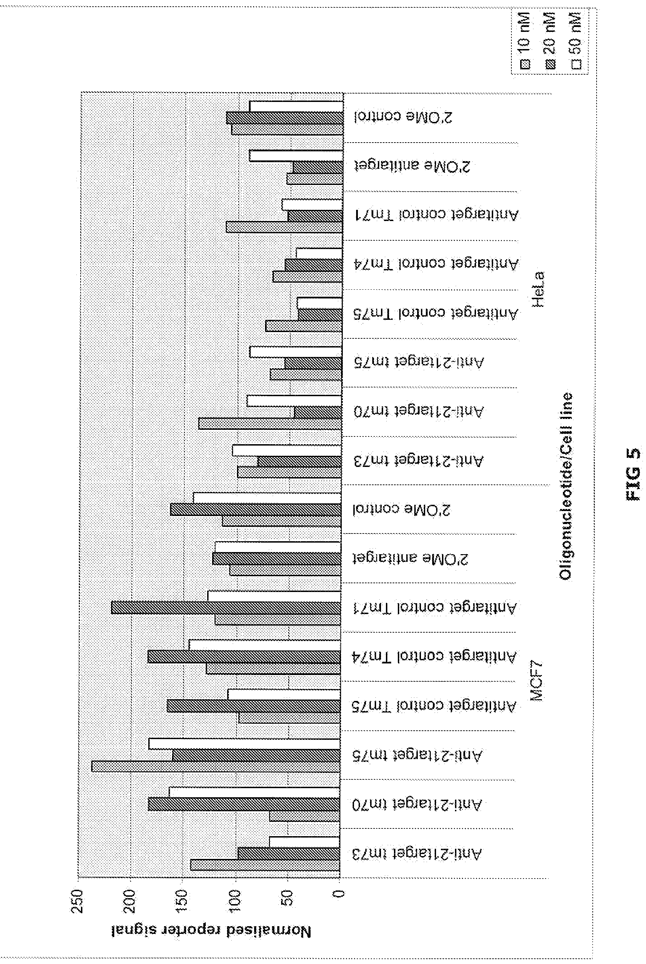

Figure 5: Target site blocking pMIR-16 "control" luciferase vector. The diagram shows the normalised expression level of the pMIR-16 reporter vector as a function of oligonucleotide concentration. The left panel shows results obtained from HeLa cells and the right panel results obtained from MCF7 cells.

### Definitions

For the purposes of the subsequent detailed description of the invention the following definitions are provided for specific terms, which are used in the disclosure of the present invention:
In the present context, the terms "blocking oligo" or "blocking molecule" refer to an oligonucleotide, which comprises a recognition sequence partly complementary to the target site of a microRNA.

"miRNA target site" or "microRNA target site" refers to a specific target binding sequence of a microRNA in a mRNA target. Complementarity between the miRNA and its target site need not be perfect.

In the present context "ligand" means something that binds. Ligands include biotin and functional groups such as: aromatic groups (such as benzene, pyridine, naphtalene, anthracene, and phenanthrene), heteroaromatic groups (such as thiophene, furan, tetrahydrofuran, pyridine, dioxane, and pyrimidine), carboxylic acids, carboxylic acid esters, carboxylic acid halides, carboxylic acid azides, carboxylic acid hydrazides, sulfonic acids, sulfonic acid esters, sulfonic acid halides, semi-carbazides, thiosemicarbazides, aldehydes, ketones, primary alcohols, secondary alcohols, tertiary alcohols, phenols, alkyl halides, thiols, disulphides, primary amines, secondary amines, tertiary amines, hydrazines, epoxides, maleimides, C₁-C₂₀ alkyl groups optionally interrupted or terminated with one or more heteroatoms such as oxygen atoms, nitrogen atoms, and/or sulphur atoms, optionally containing aromatic or mono/polyunsaturated hydrocarbons, polyoxyethylene such as polyethylene glycol, oligo/polyamides such as poly-β-alanine, polyglycine, polylysine, peptides, oligo/polysaccharides, oligo/polyphosphates, toxins, antibiotics, cell poisons, and steroids, and also "affinity ligands", *i.e.,* functional groups or biomolecules that have a specific affinity for sites on particular proteins, antibodies, poly- and oligosaccharides, and other biomolecules.

The singular form "a", "an" and "the" include plural references unless the context clearly dictates otherwise. For example, the term "a cell" includes a plurality of cells, including mixtures thereof. The term "a nucleic acid molecule" includes a plurality of nucleic acid molecules.

"Transcriptome" refers to the complete collection of transcriptional units of the genome of any species. In addition to protein-coding mRNAs, it also represents non-coding RNAs, such as microRNAs, which have important structural and regulatory roles in the cell.

"Sample" refers to a sample of cells, or tissue or fluid isolated from an organism or organisms, including but not limited to, for example, skin, plasma, serum, spinal fluid, lymph fluid, synovial fluid, urine, tears, blood cells, organs, tumours, and also to samples of *in vitro* cell culture constituents (including but not limited to conditioned medium resulting from the growth of cells in cell culture medium, recombinant cells and cell components).

An "organism" refers to an entity alive at some time, including but not limited to, for example, human, mouse, rat, *Drosophila, C. elegans,* yeast, *Arabidopsis thaliana,* maize, rice, zebra fish, primates, domestic animals, etc.

The terms "detection probe" or "detection probe sequence" refer to an oligonucleotide including a recognition sequence complementary to a RNA target sequence, wherein the recognition sequence is substituted with a high-affinity nucleotide analogs, e.g., LNA, to increase the sensitivity and specificity compared to conventional oligonucleotides, such as DNA oligonucleotides, for hybridization to short target sequences, e.g., mature miRNAs, stem-loop precursor miRNAs, pri-miRNAs, as well as miRNA binding sites in their cognate mRNA targets.

The terms "miRNA" and "microRNA" refer to 21-25 nt non-coding RNAs derived from endogenous genes. They are processed from longer (ca. 75 nt) hairpin-like precursors termed pre-miRNAs. MicroRNAs assemble in complexes termed miRNPs and recognize their targets by antisense complementarity. If the microRNAs match 100 % their target, i.e., the complementarity is complete, the target mRNA is cleaved, and the miRNA acts like a siRNA. If the match is incomplete, i.e., the complementarity is partial, then the translation of the target mRNA is blocked.

The term "recognition sequence" refers to a nucleotide sequence that is complementary to a region within the target nucleotide sequence essential for sequence-specific hybridization between the target nucleotide sequence and the recognition sequence.

The term "label" as used herein refers to any atom or molecule which can be used to provide a detectable (preferably quantifiable) signal, and which can be attached to a nucleic acid or protein. Labels may provide signals detectable by fluorescence, radioactivity, colorimetric, X-ray diffraction or absorption, magnetism, enzymatic activity, and the like.

As used herein, the terms "nucleic acid", "polynucleotide" and "oligonucleotide" refer to primers, probes, oligomer fragments to be detected, oligomer controls and unlabelled blocking oligomers and shall be generic to polydeoxyribonucleotides (containing 2-deoxy-D-ribose), to polyribonucleotides (containing D-ribose), and to any other type of polynucleotide which is an N glycoside of a nucleobase, e.g., purine or pyrimidine base, or modified purine or pyrimidine bases. There is no intended distinction in length between the term "nucleic acid", "polynucleotide" and "oligonucleotide", and these terms will be used interchangeably. These terms refer only to the primary structure of the molecule. Thus, these terms include double- and single-stranded DNA, as well as double- and single stranded RNA. The oligonucleotide is comprised of a sequence of approximately at least 3 nucleotides, preferably at least about 6 nucleotides, and more preferably at least about 8 - 30 nucleotides corresponding to a region of the designated target nucleotide sequence. "Corresponding" means identical to or complementary to the designated sequence. The oligonucleotide is not necessarily physically derived from any existing or natural sequence but may be generated in any manner, including chemical synthesis, DNA replication, reverse transcription or a combination thereof.

The terms "oligonucleotide" or "nucleic acid" intend a polynucleotide of genomic DNA or RNA, cDNA, semi synthetic, or synthetic origin which, by virtue of its origin or manipulation: (1) is not associated with all or a portion of the polynucleotide with which it is associated in nature; and/or (2) is linked to a polynucleotide other than that to which it is linked in nature; and/or (3) is not found in nature. Because mononucleotides are reacted to make oligonucleotides in a manner such that the 5'-phosphate of one mononucleotide pentose ring is attached to the 3' oxygen of its neighbour in one direction via a phosphodiester linkage, an end of an oligonucleotide is referred to as the "5' end" if its 5' phosphate is not linked to the 3' oxygen of a mononucleotide pentose ring and as the "3' end" if its 3' oxygen is not linked to a 5' phosphate of a subsequent mononucleotide pentose ring. As used herein, a nucleic acid sequence, even if internal to a larger oligonucleotide, also may be said to have a 5' and 3' ends. When two different, non-overlapping oligonucleotides anneal to different regions of the same linear complementary nucleic acid sequence, the 3' end of one oligonucleotide points toward the 5' end of the other; the former may be called the "upstream" oligonucleotide and the latter the "downstream" oligonucleotide.

The linkage between two successive monomers in a nucleic acid consists of 2 to 4, desirably 3, groups/atoms selected from -CH₂-, -O-, -S-, -NR^{H}-, >C=O, >C=NR^{H}, >C=S, -Si(R")₂-, -SO-, -S(O)₂-, -P(O)₂-, -PO(BH₃)-, -P(O,S)-, -P(S)₂-, -PO(R")-, -PO(OCH₃)-, and -PO(NHR^{H})-, where R^{H} is selected from hydrogen and C₁₋₄-alkyl, and R" is selected from C₁₋₆-alkyl and phenyl. Illustrative examples of such linkages are -CH₂-CH₂-CH₂-, -CH₂-CO-CH₂-, -CH₂-CHOH-CH₂-, -O-CH₂-O-, -O-CH₂-CH₂-, -O-CH₂-CH= (including R⁵ when used as a linkage to a succeeding monomer), -CH₂-CH₂-O-, -NR^{H}-CH₂-CH₂-, -CH₂-CH₂-NR^{H}-, -CH₂-NR^{H}-CH₂-, -O-CH₂-CH₂-NR^{H}-, -NR^{H}-CO-O-, -NR^{H}-CO-NR^{H}-, -NR^{H}-CS-NR^{H}-, -NR^{H}-C(=NR^{H})-NR^{H}-, -NR^{H}-CO-CH₂-NR^{H}-, -O-CO-O-, -O-CO-CH₂-O-, -O-CH₂-CO-O-, -CH₂-CO-NR^{H}-, -O-CO-NR^{H}-, -NR^{H}-CO-CH₂-, -O-CH₂-CO-NR^{H}-, -O-CH₂-CH₂-NR^{H}-, -CH=N-O-, -CH₂-NR^{H}-O-, -CH₂-O-N= (including R⁵ when used as a linkage to a succeeding monomer), -CH₂-O-NR^{H}-, -CO-NR^{H}-CH₂-, -CH₂-NR^{H}-O-, -CH₂-NR^{H}-CO-, -O-NR^{H}-CH₂-, -O-NR^{H}-, -O-CH₂-S-, -S-CH₂-O-, -CH₂-CH₂-S-, -O-CH₂-CH₂-S-, -S-CH₂-CH= (including R⁵ when used as a linkage to a succeeding monomer), -S-CH₂-CH₂-, -S-CH₂-CH₂-O-, -S-CH₂-CH₂-S-, -CH₂-S-CH₂-, -CH₂-SO-CH₂-, -CH₂-SO₂-CH₂-, -O-SO-O-, -O-S(O)₂-O-, -O-S(O)₂-CH₂-, -O-S(O)₂-NR^{H}-, -NR^{H}-S(O)₂-CH₂-, -O-S(O)₂-CH₂-, -O-P(O)₂-O-, -O-P(O,S)-O-, -O-P(S)₂-O-, -S-P(O)₂-O-, -S-P(O,S)-O-, -S-P(S)₂-O-, -O-P(O)₂-S-, -O-P(O,S)-S-, -O-P(S)₂-S-, -S-P(O)₂-S-, -S-P(O,S)-S-, -S-P(S)₂-S-, -O-PO(R")-O-, -O-PO(OCH₃)-O-, -O-PO(OCH₂CH₃)-O-, -O-PO(OCH₂CH₂S-R)-O-, -O-PO(BH₃)-O-, -O-PO(NHR^{N})-O-, -O-P(O)₂-NR^{H}-, -NR^{H}-P(O)₂-O-, -O-P(O,NR^{H})-O-, -CH₂-P(O)₂-O-, -O-P(O)₂-CH₂-, and -O-Si(R")₂-O-; among which -CH₂-CO-NR^{H}-, -CH₂-NR^{H}-O-, -S-CH₂-O-, -O-P(O)₂-O-, -O-P(O,S)-O-, -O-P(S)₂-O-, -NR^{H}-P(O)₂-O-,-O-P(O,NR^{H})-O-, -O-PO(R")-O-, -O-PO(CH₃)-O-, and -O-PO(NHR^{N})-O-, where R^{H} is selected form hydrogen and C₁₋₄-alkyl, and R" is selected from C₁₋₆-alkyl and phenyl, are especially desirable. Further illustrative examples are given in Mesmaeker et. al., Current Opinion in Structural Biology 1995, 5, 343-355 and Susan M. Freier and Karl-Heinz Altmann, Nucleic Acids Research, 1997, vol 25, pp 4429-4443. The left-hand side of the internucleoside linkage is bound to the 5-membered ring as substituent P^{*} at the 3'-position, whereas the right-hand side is bound to the 5'-position of a preceding monomer.

By the term "SBC nucleobases" is meant "Selective Binding Complementary" nucleobases, i.e., modified nucleobases that can make stable hydrogen bonds to their complementary nucleobases, but are unable to make stable hydrogen bonds to other SBC nucleobases. As an example, the SBC nucleobase A', can make a stable hydrogen bonded pair with its complementary unmodified nucleobase, T. Likewise, the SBC nucleobase T' can make a stable hydrogen bonded pair with its complementary unmodified nucleobase, A. However, the SBC nucleobases A' and T' will form an unstable hydrogen bonded pair as compared to the base pairs A'-T and A-T'. Likewise, a SBC nucleobase of C is designated C' and can make a stable hydrogen bonded pair with its complementary unmodified nucleobase G, and a SBC nucleobase of G is designated G' and can make a stable hydrogen bonded pair with its complementary unmodified nucleobase C, yet C' and G' will form an unstable hydrogen bonded pair as compared to the base pairs C'-G and C-G'. A stable hydrogen bonded pair is obtained when 2 or more hydrogen bonds are formed e.g. the pair between A' and T, A and T', C and G', and C' and G. An unstable hydrogen bonded pair is obtained when 1 or no hydrogen bonds is formed e.g. the pair between A' and T', and C' and G'. Especially interesting SBC nucleobases are 2,6-diaminopurine (A', also called D) together with 2-thio-uracil (U', also called ^{2S}U)(2-thio-4-oxo-pyrimidine) and 2-thio-thymine (T', also called ^{2S}T)(2-thio-4-oxo-5-methyl-pyrimidine). The pairs A-^{2S}T and D-T have 2 or more than 2 hydrogen bonds whereas the D-^{2S}T pair forms a single (unstable) hydrogen bond. Likewise SBC nucleobases include pyrrolo-[2,3-d]pyrimidine-2(3H)-one (C', also called PyrroloPyr) and hypoxanthine (G', also called l)(6-oxo-purine), where the pairs PyrroloPyr-G and C-I have 2 hydrogen bonds each whereas the PyrroloPyr-I pair forms a single hydrogen bond.

"SBC LNA oligomer" refers to a "LNA oligomer" containing at least one LNA monomer where the nucleobase is a "SBC nucleobase". Generally speaking SBC LNA oligomers include oligomers that besides the SBC LNA monomer(s) contain other modified or naturally occurring nucleotides or nucleosides. By "SBC monomer" is meant a non-LNA monomer with a SBC nucleobase. By "isose-quential oligonucleotide" is meant an oligonucleotide with the same sequence in a Watson-Crick sense as the corresponding modified oligonucleotide e.g. the sequences agTtcATg is equal to agTscD^{2S}Ug where s is equal to the SBC DNA monomer 2-thio-t or 2-thio-u, D is equal to the SBC LNA monomer LNA-D, and ^{2S}U is equal to the SBC LNA monomer LNA ^{2S}U.

The complement of a nucleic acid sequence as used herein refers to an oligonucleotide which, when aligned with the nucleic acid sequence such that the 5' end of one sequence is paired with the 3' end of the other, is in "antiparallel association." Bases not commonly found in natural nucleic acids that may be included in the nucleic acids of the present invention include, for example, inosine and 7-deazaguanine. Complementarity may not be perfect; stable duplexes may contain mismatched base pairs or unmatched bases. Those skilled in the art of nucleic acid technology can determine duplex stability empirically considering a number of variables including, for example, the length of the oligonucleotide, percent concentration of cytosine and guanine bases in the oligonucleotide, ionic strength, and incidence of mismatched base pairs.

Stability of a nucleic acid duplex is measured by the melting temperature, or "Tₘ". The Tₘ of a particular nucleic acid duplex under specified conditions is the temperature at which half of the duplexes have disassociated. Stability can also be used as a measure of binding affinity of an oligonucleotide towards its target.

The term "nucleobase" covers the naturally occurring nucleobases adenine (A), guanine (G), cytosine (C), thymine (T) and uracil (U) as well as non-naturally occurring nucleobases such as xanthine, diaminopurine, 8-oxo-N⁶-methyladenine, 7-deazaxanthine, 7-deazaguanine, N⁴,N⁴-ethanocytosin, N⁶,N⁶-ethano-2,6-diaminopurine, 5-methylcytosine, 5-(C³-C⁶)-alkynyl-cytosine, 5-fluorouracil, 5-bromouracil, pseudoisocytosine, 2-hydroxy-5-methyl-4-triazolopyridin, isocytosine, isoguanine, inosine and the "non-naturally occurring" nucleobases described in Benner et al., U.S. Patent No. 5,432,272 and Susan M. Freier and Karl-Heinz Altmann, Nucleic Acid Research,25: 4429-4443, 1997. The term "nucleobase" thus includes not only the known purine and pyrimidine heterocycles, but also heterocyclic analogues and tautomers thereof. Further naturally and non naturally occurring nucleobases include those disclosed in U.S. Patent No. 3,687,808; in chapter 15 by Sanghvi, in Antisense Research and Application, Ed. S. T. Crooke and B. Lebleu, CRC Press, 1993; in Englisch, et al., Angewandte Chemie, International Edition, 30: 613-722, 1991 (see, especially pages 622 and 623, and in the Concise Encyclopedia of Polymer Science and Engineering, J. I. Kroschwitz Ed., John Wiley & Sons, pages 858-859, 1990, Cook, Anti-Cancer DrugDesign 6: 585-607, 1991).

The term "nucleobase" is further intended to include heterocyclic compounds that can serve as like nucleosidic bases including certain "universal bases" that are not nucleosidic bases in the most classical sense but serve as nucleosidic bases. Especially mentioned as a universal base is 3-nitropyrrole or a 5-nitroindole. Other preferred compounds include pyrene and pyridyloxazole derivatives, pyrenyl, pyrenylmethylglycerol derivatives and the like. Other preferred universal bases include, pyrrole, diazole or triazole derivatives, including those universal bases known in the art.

By "LNA" or "LNA monomer" (e.g., an LNA nucleoside or LNA nucleotide) is meant a nucleoside or nucleotide analogue that includes at least one LNA monomer. LNA monomers as disclosed in PCT Publication WO 99/14226 are in general particularly desirable modified nucleic acids for incorporation into an oligonucleotide of the invention. Additionally, the nucleic acids may be modified at either the 3' and/or 5' end by any type of modification known in the art. For example, either or both ends may be capped with a protecting group, attached to a flexible linking group, attached to a reactive group to aid in attachment to the substrate surface, etc. Desirable LNA monomers and their method of synthesis also are disclosed in US 6,043,060, US 6,268,490, PCT Publications WO 01/07455, WO 01/00641, WO 98/39352, WO 00/56746, WO 00/56748 and WO 00/66604 as well as in the following papers: Morita et al., Bioorg. Med. Chem. Lett. 12(1):73-76, 2002; Hakansson et al., Bioorg. Med. Chem. Lett. 11(7):935-938, 2001; Koshkin et al., J. Org. Chem. 66(25):8504-8512, 2001; Kvaerno et al., J. Org. Chem. 66(16):5498-5503, 2001; Hakansson et al., J. Org. Chem. 65(17):5161-5166, 2000; Kvaerno et al., J. Org. Chem. 65(17):5167-5176, 2000; Pfundheller et al., Nucleosides Nucleotides 18(9):2017-2030, 1999; and Kumar et al., Bioorg. Med. Chem. Lett. 8(16):2219-2222, 1998.

Preferred LNA monomers, also referred to as "oxy-LNA" are LNA monomers which include bicyclic compounds as disclosed in PCT Publication WO 03/020739 wherein the bridge between R^{4'} and R^{2'} as shown in formula (I) below together designate -CH₂-O- or -CH₂-CH₂-O-.

By "LNA modified oligonucleotide" or "LNA substituted oligonucleotide" is meant an oligonucleotide comprising at least one LNA monomer of formula (I), described *infra,* having the below described illustrative examples of modifications: 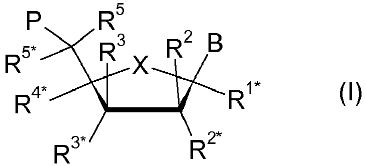 wherein X is selected from -O-, -S-, -N(R^{N})-, -C(R⁶R^{6*})-, -O-C(R⁷R^{7*})-, -C(R⁶R^{6*})-O-, -S-C(R⁷R^{7*})-, -C(R⁶R^{6*})-S-, -N(R^{N*})-C(R⁷R^{7*})-, -C(R⁶R^{6*})-N(R^{N*})-, and -C(R⁶R^{6*})-C(R⁷R^{7*}).

B is selected from a modified base as discussed above e.g. an optionally substituted carbocyclic aryl such as optionally substituted pyrene or optionally substituted pyrenylmethylglycerol, or an optionally substituted heteroalicylic or optionally substituted heteroaromatic such as optionally substituted pyridyloxazole, optionally substituted pyrrole, optionally substituted diazole or optionally substituted triazole moieties; hydrogen, hydroxy, optionally substituted C₁₋₄-alkoxy, optionally substituted C₁₋₄-alkyl, optionally substituted C₁₋₄-acyloxy, nucleobases, DNA intercalators, photochemically active groups, thermochemically active groups, chelating groups, reporter groups, and ligands.

P designates the radical position for an internucleoside linkage to a succeeding monomer, or a 5'-terminal group, such internucleoside linkage or 5'-terminal group optionally including the substituent R⁵. One of the substituents R², R^{2*}, R³, and R^{3*} is a group P^{*} which designates an internucleoside linkage to a preceding monomer, or a 2'/3'-terminal group. The substituents of R^{1*}, R^{4*}, R⁵, R^{5*}, R⁶, R^{6*}, R⁷, R^{7*}, R^{N}, and the ones of R², R^{2*}, R³, and R^{3*} not designating P^{*} each designates a biradical comprising about 1-8 groups/atoms selected from -C(R^{a}R^{b})-, -C(R^{a})=C(R^{a})-, -C(R^{a})=N-, -C(R^{a})-O-, -O-, -Si(R^{a})₂-, -C(R^{a})-S, -S-, -SO₂-, -C(R^{a})-N(R^{b})-, -N(R^{a})-, and >C=Q, wherein Q is selected from -O-, -S-, and -N(R^{a})-, and R^{a} and R^{b} each is independently selected from hydrogen, optionally substituted C₁₋₁₂-alkyl, optionally substituted C₂₋₁₂-alkenyl, optionally substituted C₂₋₁₂-alkynyl, hydroxy, C₁₋₁₂-alkoxy, C₂₋₁₂-alkenyloxy, carboxy, C₁₋₁₂-alkoxycarbonyl, C₁₋₁₂-alkylcarbonyl, formyl, aryl, aryloxy-carbonyl, aryloxy, arylcarbonyl, heteroaryl, hetero-aryloxy-carbonyl, heteroaryloxy, heteroarylcarbonyl, amino, mono- and di(C₁₋₆-alkyl)amino, carbamoyl, mono- and di(C₁₋₆-alkyl)-aminocarbonyl, amino-C₁₋₆-alkyl-aminocarbonyl, mono- and di(C₁₋₆-alkyl)amino-C₁₋₆-alkyl-aminocarbonyl, C₁₋₆-alkyl-carbonylamino, carbamido, C₁₋₆-alkanoyloxy, sulphono, C₁₋₆-alkylsulphonyloxy, nitro, azido, sulphanyl, C₁₋₆-alkylthio, halogen, DNA intercalators, photochemically active groups, thermochemically active groups, chelating groups, reporter groups, and ligands, where aryl and heteroaryl may be optionally substituted, and where two geminal substituents R^{a} and R^{b} together may designate optionally substituted methylene (=CH₂), and wherein two non-geminal or geminal substituents selected from R^{a}, R^{b}, and any of the substituents R^{1*}, R², R^{2*}, R³, R^{3*}, R^{4*}, R⁵, R^{5*}, R⁶ and R^{6*}, R⁷, and R^{7*} which are present and not involved in P, P^{*} or the biradical(s) together may form an associated biradical selected from biradicals of the same kind as defined before; the pair(s) of non-geminal substituents thereby forming a mono- or bicyclic entity together with (i) the atoms to which said non-geminal substituents are bound and (ii) any intervening atoms.

Each of the substituents R^{1*}, R², R^{2*}, R³, R^{4*}, R⁵, R^{5*}, R⁶ and R^{6*}, R⁷, and R^{7*} which are present and not involved in P, P^{*} or the biradical(s), is independently selected from hydrogen, optionally substituted C₁₋₁₂-alkyl, optionally substituted C₂₋₁₂-alkenyl, optionally substituted C₂₋₁₂-alkynyl, hydroxy, C₁₋₁₂-alkoxy, C₂₋₁₂-alkenyloxy, carboxy, C₁₋₁₂-alkoxycarbonyl, C₁₋₁₂-alkylcarbonyl, formyl, aryl, aryloxy-carbonyl, aryloxy, arylcarbonyl, heteroaryl, heteroaryloxy-carbonyl, heteroaryloxy, heteroarylcarbonyl, amino, mono- and di-(C₁₋₆-alkyl)amino, carbamoyl, mono- and di(C₁₋₆-alkyl)-amino-carbonyl, amino-C₁₋₆-alkyl-aminocarbonyl, mono- and di(C₁₋₆-alkyl)amino-C₁₋₆-alkyl-aminocarbonyl, C₁₋₆-alkylcarbonylamino, carbamido, C₁₋₆-alkanoyloxy, sulphono, C₁₋₆-alkylsulphonyloxy, nitro, azido, sulphanyl, C₁₋₆-alkylthio, halogen, DNA intercalators, photochemically active groups, thermochemically active groups, chelating groups, reporter groups, and ligands, where aryl and heteroaryl may be optionally substituted, and where two geminal substituents together may designate oxo, thioxo, imino, or optionally substituted methylene, or together may form a spiro biradical consisting of a 1-5 carbon atom(s) alkylene chain which is optionally interrupted and/or terminated by one or more heteroatoms/groups selected from -O-, -S-, and -(NR^{N})- where R^{N} is selected from hydrogen and C₁₋₄-alkyl, and where two adjacent (non-geminal) substituents may designate an additional bond resulting in a double bond; and R^{N*}, when present and not involved in a biradical, is selected from hydrogen and C₁₋₄-alkyl; and basic salts and acid addition salts thereof.

Exemplary 5', 3', and/or 2' terminal groups include -H, -OH, halo (*e.g.*, chloro, fluoro, iodo, or bromo), optionally substituted aryl, (*e.g.*, phenyl or benzyl), alkyl (*e.g*., methyl or ethyl), alkoxy (*e.g.*, methoxy), acyl (*e.g.* acetyl or benzoyl), aroyl, aralkyl, hydroxy, hydroxyalkyl, alkoxy, aryloxy, aralkoxy, nitro, cyano, carboxy, alkoxycarbonyl, aryloxycarbonyl, aralkoxycarbonyl, acylamino, aroylamino, alkylsulfonyl, arylsulfonyl, heteroarylsulfonyl, alkylsulfinyl, arylsulfinyl, heteroarylsulfinyl, alkylthio, arylthio, heteroarylthio, aralkylthio, heteroaralkylthio, amidino, amino, carbamoyl, sulfamoyl, alkene, alkyne, protecting groups (e.g., silyl, 4,4'-dimethoxytrityl, monomethoxytrityl, or trityl(triphenylmethyl)), linkers (e.g., a linker containing an amine, ethylene glycol, quinone such as anthraquinone), detectable labels (e.g., radiolabels or fluorescent labels), and biotin.

It is understood that references herein to a nucleic acid unit, nucleic acid residue, LNA monomer, or similar term are inclusive of both individual nucleoside units and nucleotide units and nucleoside units and nucleotide units within an oligonucleotide.

A "modified base" or other similar terms refer to a composition (e.g., a non-naturally occurring nucleobase or nucleosidic base), which can pair with a natural base (e.g., adenine, guanine, cytosine, uracil, and/or thymine) and/or can pair with a non-naturally occurring nucleobase or nucleosidic base. Desirably, the modified base provides a Tₘ differential of 15, 12, 10, 8, 6, 4, or 2°C or less as described herein. Exemplary modified bases are described in EP 1 072 679 and WO 97/12896.

The term "chemical moiety" refers to a part of a molecule. "Modified by a chemical moiety" thus refer to a modification of the standard molecular structure by inclusion of an unusual chemical structure. The attachment of said structure can be covalent or non-covalent.

The term "inclusion of a chemical moiety" in an oligonucleotide probe thus refers to attachment of a molecular structure. Such as chemical moiety include but are not limited to covalently and/or non-covalently bound minor groove binders (MGB) and/or intercalating nucleic acids (INA) selected from a group consisting of asymmetric cyanine dyes, DAPI, SYBR Green I, SYBR Green II, SYBR Gold, PicoGreen, thiazole orange, Hoechst 33342, Ethidium Bromide, 1-O-(1-pyrenylmethyl)glycerol and Hoechst 33258. Other chemical moieties include the modified nucleobases, nucleosidic bases or LNA modified oligonucleotides.

"Oligonucleotide analog" refers to a nucleic acid binding molecule capable of recognizing a particular target nucleotide sequence. A particular oligonucleotide analogue is peptide nucleic acid (PNA) in which the sugar phosphate backbone of an oligonucleotide is replaced by a protein like backbone. In PNA, nucleobases are attached to the uncharged polyamide backbone yielding a chimeric pseudopeptide-nucleic acid structure, which is homomorphous to nucleic acid forms.

"High affinity nucleotide analogue" refers to a non-naturally occurring nucleotide analogue that increases the "binding affinity" of an oligonucleotide probe to its complementary recognition sequence when substituted with at least one such high-affinity nucleotide analogue. Commonly used analogues include 2'-O-methyl-modified nucleic acids (2'-OMe) (RNA, 2006, 12, 163-176), 2'-O-(2-methoxyethyl)-modified nucleic acids (2'-MOE) (Nucleic Acids Research, 1998, 26, 16, 3694-3699), 2'-Deoxy-2'-fluoro-β-D-arabinoic acid (FANA) (Nucleic Acids Research, 2006, 34, 2, 451-461), Cyclohexene nucleic acids (CeNA) (Nucleic Acids Research, 2001, 29, 24, 4941-4947), Hexitol nucleic acids (HNA) and analogs hereof (Nucleic Acids Research, 2001, 29, 20, 4187-4194), Intercalating Nucleic Acids (INA) (Helvetica Chimica Acta, 2003, 86, 2090-2097) and 2'-O,4'-C-Ethylene-bridged-Nucleic Acids (ENA) (Bioorganic and Medicinal Chemistry Letters, 2002, 12, 1, 73-76). Additionally, in the present context, the oligonucleotide mimic referred to as peptide nucleic acid (PNA) (Nielsen et al., Science 254; 1497-1500, 1991 and U.S. Pat. Nos. 5,539,082; 5,714,331; and 5,719,262) is considered a high affinity nucleotide analogue. A preferred high affinity nuceltodie analogue is LNA. A plurality of a combination of analogues may also be employed in an oligo of the invention.

As used herein, an oligo with an increased "binding affinity" for a recognition sequence compared to an oligo that includes the same sequence but does not include a nucleotide analog, refers to an oligo for which the association constant (Kₐ) of the recognition segment is higher than the association constant of the complementary strands of a double-stranded molecule. In a preferred embodiment, the association constant of the recognition segment is higher than the dissociation constant (K_{d}) of the complementary strand of the recognition sequence in the target sequence in a double stranded molecule.

Monomers are referred to as being "complementary" if they contain nucleobases that can form hydrogen bonds according to Watson-Crick base-pairing rules (e.g. G with C, A with T or A with U) or other hydrogen bonding motifs such as for example diaminopurine with T, 5-methyl C with G, 2-thiothymidine with A, inosine with C, pseudoisocytosine with G, etc.

The term "succeeding monomer" relates to the neighbouring monomer in the 5'-terminal direction and the "preceding monomer" relates to the neighbouring monomer in the 3'-terminal direction.

The term "target nucleic acid" or "target ribonucleic acid" refers to any relevant nucleic acid of a single specific sequence, e.g., a biological nucleic acid, e.g., derived from a patient, an animal (a human or non-human animal), a plant, a bacteria, a fungi, an archae, a cell, a tissue, an organism, etc. For example, where the target ribonucleic acid or nucleic acid is derived from a bacteria, archae, plant, non-human animal, cell, fungi, or non-human organism, the method optionally further comprises selecting the bacteria, archae, plant, non-human animal, cell, fungi, or non-human organism based upon detection of the target nucleic acid. In one embodiment, the target nucleic acid is derived from a patient, e.g., a human patient. In this embodiment, the invention optionally further includes selecting a treatment, diagnosing a disease, or diagnosing a genetic predisposition to a disease, based upon detection of the target nucleic acid.

"Target sequence" refers to a specific nucleic acid sequence within any target nucleic acid.

The term "stringent conditions", as used herein, is the "stringency" which occurs within a range from about Tₘ-5° C. (5° C. below the melting temperature (Tₘ) of the probe) to about 20° C. to 25° C. below Tₘ. As will be understood by those skilled in the art, the stringency of hybridization may be altered in order to identify or detect identical or related polynucleotide sequences. Hybridization techniques are generally described in Nucleic Acid Hybridization, A Practical Approach, Ed. Hames, B. D. and Higgins, S. J., IRL Press, 1985; Gall and Pardue, Proc. Natl. Acad. Sci., USA 63: 378-383, 1969; and John, et al. Nature 223: 582-587, 1969.

### Detailed Description of the Invention

MicroRNAs target mRNA sequences in a sequence specific manner by inducing mRNA degradation or inhibiting protein synthesis by blocking translation. It has recently been demonstrated that each microRNA may have multiple targets, in some cases up to several hundreds. To study the effects of microRNAs one option is to introduce exogenous microRNAs as oligonucleotides or expressed from transfected expression constructs and analyse the effect on target gene or protein expression. Another option is to introduce an oligonucleotide complementary to the microRNA or antisense molecule, in this way blocking the effect of the microRNA. However, both these options will either potentially induce or block the effect on multiple genes, that all have the target sequence for the microRNA.

It is therefore desirable to have available reagents that can specifically block the effect of individual microRNAs or antisense molecules on single genes, rather than addressing multiple genes simultaneously. One way to achieve this is by transfecting cells with nucleic acid constructs that are homologous to the target sequence of the microRNA or antisense molecule and are able to block this site thereby making it inaccessible to the regulatory microRNA or antisense molecule. However, it is of utmost importance that the blocking nucleic acid molecule can bind with high affinity compared to microRNA or antisense molecules, that it has high nuclease resistance, and very importantly that it does not induce antisense (e.g., RNAseH induction) effect on the target molecule.

High affinity nucleic acid analog (e.g., LNA) containing molecules have several advantages for this purpose:
- nucleic acid analogs can increase thermal stability allowing the blocking molecule to bind preferentially to the target site of the microRNA or antisense molecule, preferably to the microRNA or antisense molecule itself.
- nucleic acid analog containing molecules show increased stability compared to normal nucleic acid molecules either DNA or RNA
- it has been shown that when employing nucleotide analogues such as LNAs for antisense molecules, a 5-8 nucleotide centrally located "gap" of un-modified DNA or RNA molecules is necessary to induce RNAseH mediated degradation of the target (Kurreck et al., Nucleic Acids Res. 30(9); 1911-1918, 2002).
- Furthermore it has been demonstrated that LNA does not induce interferon response in in vivo administration.

Thus, nucleic acid molecules, which do not induce antisense (e.g., RNAseH induction) effects on the target molecule can be designed to block regulatory target sites for microRNA or antisense molecules.

When designing nucleic acid constructs that are homologous to the target sequence of the microRNA or antisense molecule and are able to block this site by making it inaccessible to the microRNA or antisense molecule, it is important to acknowledge, that a large part of the microRNA target sequence is conserved among multiple targets in the transcriptome. Recent work from Brennecke et al. (PloS Biol 3(3): e85, 2005) has provided evidence that an average miRNA has approximately 100 target sites, indicating that miRNAs regulate a large fraction of protein-coding genes. Hence, if the blocking oligonucleotide is targeted against the precise target sequence of the specific gene transcript, it may or may not also block target sequences at other genes, which may not be desirable.

Target sites can be grouped into two broad categories having primarily 5'end or 3'end complementarity to the target transcript. 5' dominant sites have sufficient complementarity to the miRNA 5' end to function with little or no support from pairing to the miRNA 3' end. Indeed, sites with 3' pairing below the random noise level are functional given a strong 5' end. In contrast, 3' compensatory sites have insufficient 5' pairing and require strong 3' pairing for function. miRNA 3' ends are key determinants of target specificity within miRNA families.

Thus, one advantageous principle of designing target site blocking nucleic acids is to design an oligonucleotide overlapping the 5' or 3' end of the microRNA target site, and at the same time partly overlapping non-microRNA target sites. This would both block the accessibility of the microRNA and provide transcript specificity to the anti-MIR oligo.

In a presently preferred embodiment, the present invention relates to a method for producing a microRNA target site blocking nucleic acid directed at a specific transcript, wherein an oligonucleotide sequence of the nucleic acid overlaps the 5' or 3' end of the microRNA target site, and at the same time partly overlaps the non-microRNA target sites, the method comprising the steps of:
1) selecting a known microRNA having at least one identified or putative microRNA target site,
2) obtaining the sequence of said microRNA and the sequence of one of said microRNA target sites,
3) selecting and obtaining the sequence of a transcript which comprises the microRNA target site selected in step 2,
4) designing a microRNA target site blocking nucleic acid which is at least 10 nucleotides long and comprises at least one LNA analogue and which is either
   a) at least partially complementary to the microRNA target site and which comprises a consecutive sequence of nucleotides which are complementary to the 3' end of the microRNA target site selected in step 3 and a consecutive sequence of nucleotides which are complementary to the transcript sequence immediately adjacent and downstream of the 3' end of the microRNA target site, or
   b) at least partially complementary to the microRNA target site and which comprises a consecutive sequence of nucleotides which are complementary to end 5' end of the microRNA target site selected in step 3 and a consecutive sequence of nucleotides which are complementary to the transcript sequence immediately adjacent and upstream of the 5' end of the microRNA target site
5) synthesizing the nucleic acid designed in step 4.

Another advantageous design principle would be to include, at least, interspaced nucleic acid analogues in the entire sequence, to prevent the formation of gapmers. Gaps should not exceed 4 nucleotides.

Yet another advantageous design feature would be to include in the blocking nucleic acid nucleic acid analogues in the 3' and 5' ends to enhance bio-stability and to decrease liability to intracellular nucleases.

Examples of blocking oligos for a microRNA target site for human mir has-let-7a in the gene transcript target gene ENSG00000107719 are depicted in figures 1A and 1B.

Exemplary miRNAs and their targets are described herein and are known in the art, e.g. in the miRBase Sequence Database (D140-D144 Nucleic Acids Research, 2006, Vol. 34, Database Issue) and miRGen (D149-D155 Nucleic Acids Research, 2006, Vol. 35, Database Issue).

### MicroRNA Target Recognition

microRNAs can bind either by perfect or partial complementarity to mRNA targets. When binding is partially complementary, fully complementary binding of the 2-8 nucleotide 5' region of the microRNA is of specific importance for functional activity of the microRNA (Bartel et al., Cell 116; 281-297, 2004). The importance of complementarity to the 5' portion of metazoan miRNAs has been suspected since the observation that the *lin-14* UTR has "core elements" of complementarity to the 5' region of the *lin-4* miRNA (Wightman et al., Cell 75; 855-862, 1993). More recent observations support this idea: (1) Residues 2-8 of several invertebrate miRNAs are perfectly complementary to 3' UTR elements previously shown to mediate posttranscriptional repression (Lai, Nature Genetics 30; 363-364, 2002). (2) Within the miRNA complementary sites of the first validated targets of invertebrate miRNAs, mRNA residues that pair (sometimes imperfectly) to residues 2-8 of the miRNA are perfectly conserved in orthologous messages of other species, and a contiguous helix of at least six basepairs is nearly always seen in this region (Stark et al., PLOS Biol 1; e60, 2003). (3) Residues 2-8 of the miRNA are the most conserved among homologous metazoan miRNAs (Lewis et al., Cell 115; 787-798, 2003; Lim et al., Genes Dev 17; 991-1008, 2003a). (4) When predicting targets of mammalian miRNAs, requiring perfect pairing to the heptamer spanning residues 2-8 of the miRNA is much more productive than is requiring pairing to any other heptamer of the miRNA (Lewis et al., Cell 115; 787-798, 2003).

### Determining and modulating the functional role of microRNAs

Prediction software often bases predictions of microRNA target sites on perfect complementarity of target 5' seed sequences and partial complementarity of the remaining sequences. Since microRNAs often elicit their effect through incomplete binding to target nucleotide sequences, bioin-formatically predicting the target nucleotides (e.g., mRNAs) of a given microRNA based on its sequence alone is not trivial and may not provide evidence that an interaction is occurring in vivo. One way of experimentally investigating the interaction between a microRNA and its target is to inactivate the microRNA in question (e.g., by providing a complementary knock-down oligo). However, each microRNA may have multiple target nucleic acids (e.g., mRNAs) in the cell, in some case more than 200 predicted targets may exist for a given microRNA. Hence, inactivating a specific microRNA in a cell may not directly provide evidence for interaction between a specific microRNA and a target nucleic acid (e.g., mRNA), since potential effects may be elicited by interactions between the microRNA and other targets in the cell.

A challenge in microRNA research is therefore to establish evidence that an interaction occurs between a microRNA and a prediction microRNA target site in a target nucleic acid (e.g., mRNA). By providing a method by which to specifically block a particular microRNA target site in a particular target nucleotide, the present invention provides a solution to study the specific interaction between a microRNA and its target.

### Modulating microRNA interactions for specific target nucleotides.

MicroRNAs have been shown to be involved in several types of diseases, as described herein, and therapeutic strategies have been contemplated where specific microRNAs are blocked or inhibited, to treat microRNA related diseases. However, as described, each microRNA may have multiple target nucleic acids (e.g., mRNAs) in the cell. Hence, inactivating a specific microRNA in a cell will not only affect the interaction between a specific microRNA and a target nucleic acid (e.g., mRNA) but will also affect interactions between the microRNA and other targets in the cell generally. In strategies to develop therapeutic agents to treat microRNA mediated diseases, a challenge in microRNA research has been therefore to establish a method to modulate an interaction that occurs between a specific microRNA and one or more specific microRNA target sites in one or more specific target nucleic acids (e.g., mRNA). By providing a method by which to specifically block a particular microRNA target site in a particular target nucleic acid, the present invention provides a solution to develop specific therapeutic agents directed against microRNA mediated diseases

### Design Parameters

Computational predictions have been the mainstay for discovery of microRNAs and their targets. Multiple computer prediction algorithms have been developed that use established miRNA-mRNA interaction rules, some of which have been trained on existing microarray data, to identify miRNA targets (John et al., PloS Biol 2; e363, 2004; Krek et al., Nature Genetics 37; 495-500, 2005; Kiriakidou et al., Genes Dev 18; 1165-1178; Lewis et al., Cell 120; 15-20, 2005; Robins and Padgett, PNAS 102;4006-4009, 2005). The algorithms used to predict miRNa targets typically develop scoring schemes based on sequence complementarity, free energy calculations of RNA duplex formation, phylogenetic conservation, and target RNA structure. Several algorithms are readily available on the web, such as TargetScan (http://genes.mit.edu/targetscan/) (Lewis et al., Cell 120; 15-20, 2005), Miranda (http://www.microrna.org) (John et al., PloS Biol 2; e363, 2004), PicTar (http://pictar.bio.nyu.edu/cgi-bin/PicTar_vertebrate.cgi) (Krek et al., Nature Genetics 37; 495-500, 2005) and DIANA-microT (http://www.diana.pcbi.upenn.edu/) (Kiriakidou et al., Genes Dev 18; 1165-1178).

One method for identification of miRNA targets relies on measuring reductions in target mRNA levels caused by an exogenously added miRNA (Lim et al., Nature 433; 769-773, 2005).

Recently, direct biochemical methods that combine RNA-induced silencing complex (RISC) purification with microarray analysis bound mRNAs have been used for miRNA target discovery (Kar-ginov et al., PNAS 104(49); 19291-19296; Easow et al., RNA 13(8); 1198-1204, 2007).

US2004/0175732 describes further methods of identifying microRNA targets, whether or not the sequence of the miRNA is known, by obtaining an miRNA/target RNA complex and transcribe target complementary RNA from the target RNA. cDNA is synthesized and the cDNA is sequenced.

Potential targets can typically be validated by using luciferase reporters containing the target 3'UTR.

Other design parameters for blocking oligos:

### Identification and validation of miRNA target sites

In order to design microRNA target site blocking oligos, functional microRNA sites needs to be identified and preferably validated, as described above.

### Blocking oligo desirably blocks miRNA target binding efficiently.

MicroRNA molecules can target mRNA sequences with both complete and incomplete homology between the miRNA sequence and target. For incomplete binding, perfect homology of the 2-8 bp so called seed sequence of the miRNA target site is of key importance for the miRNA binding and effect. The blocking oligo should preferably block at least 1-3 of the miRNA nucleotide binding events, more preferably 3-8 nucleotide binding events, also preferably selected from the seed sequence region.

To measure the effect of microRNA blocking oligonucleotides, a relative measure comparing the range between 1) the level of a given microRNA target nucleotide or resulting protein under an approximate maximum effect of a microRNA (e.g., given the natural microRNA level in a specific cell or the effect of over expression of the microRNA) and 2) the level of the microRNA target nucleotide or resulting protein without the microRNA present (e.g., in a cell not expressing the microRNA or by co-transfecting with a microRNA knockdown probe) with 3) the level of the microRNA target nucleotide or resulting protein under an approximate maximum effect of a microRNA (e.g., over expression of the microRNA) and in the presence of a given concentration of a microRNA blocking oligo targeting the same microRNA target nucleotide. For example, if the level of the microRNA target nucleotide or resulting protein is changed by 50% of the range between 1) and 2) by addition of the microRNA target site blocking oligo of a given concentration under approximate maximum effect of the microRNA, the target site blocking oligo will have blocked 50% of the microRNA effect at that given concentration.

The level of the target may be reflected in the relative expression level of the microRNA target nucleotide (e.g., a messenger RNA as determined by QPCR or northern blot or similar technologies) or in the relative expression level of the translational product (protein, as determined by e.g. Western blotting or by measuring the enzymatic or catalytic activity of the resulting protein (e.g. as lucifierase actitivty in case of the luciferase enzyme)) of the microRNA target nucleotide.

### Blocking oligo desirably only targets a single miRNA binding site, specific to a specific mRNA.

It is preferable that the blocking oligo can be designed to target only a single specific mRNA. Since each microRNA may target multiple mRNAs, target sites in different mRNAs may be very similar, hence allowing a blocking oligo designed to target one specific site, to also block other target sites. This can be avoided by designing the blocking oligo to cover part of the adjacent non-target site mRNA sequence, since this sequence will likely be specific for the mRNA in question. Furthermore, the oligo designed can be compared to a database comprising the complete transcriptome (e.g., by a BLAST search). The oligo sequence preferably should not occur more than once in such a database. Preferably, similarity with other sites differing by fewer than 2 nucleotides in identity is avoided.

### Blocking oligo desirably does not target pri- or pre-miRNA molecules to avoid blocking of endogenous miRNA production.

The sequence of the blocking oligo will be at least partially identical to the targeting miRNA sequences. Since miRNAs are produced from processed pri- and pre-miRNA molecules comprising hairpins structures involving the sequence of the mature miRNA, a miRNA target site blocking oligos comprising the complete miRNA target sequence might function to block the pre-mir and hence eliminate the production of the specific miRNA.

One microRNA can have binding sites in multiple target nucleic acids and one target sequence can be targeted by multiple microRNAs. Moreover, several binding sites for one microRNA can be found in the 3'UTR of an mRNA.

### Several miRNA binding events from the same or different miRNAs may be required to induce degradation of a specific mRNA and hence several miRNA blocking oligos may be required to protect a specific mRNA from degradation.

In one embodiment the present invention provides for the treatment of a patient with a miRNA associated disease by administering to said patient more than one oligonucleotide as described herein for blocking of more than one miRNA target sites.

### Resistance to Degradation

It has been shown (Kurreck et al., Nucleic Acids Research 30(9); 1911-1918, 2002) that LNA/DNA mixmers do not induce significant RNase H cleavage. A gap in a chimeric LNA/DNA oli-gonucletoide is needed to recruit RNase H and a DNA stretch of 7-8 nucleotides was found to provide full activation of RNase H. For gapmers with 2'-O-methyl modifications a shorter stretch of only six deoxy monomers is sufficient to induce efficient RNase H cleavage.

In a preferred embodiment, the single stranded oligonucleotide according to the invention does not mediate RNase H based cleavage of a complementary single stranded RNA molecule.

EP 1 222 309 provides in vitro methods for determining RNase H activity, which may be used to determine the ability to recruit RNase H. A compound is deemed essentially incapable of recruiting RNAse H if, when provided with the complementary RNA target, and RNase H, the RNase H initial rate, as measured in pmol/l/min, is less than 20%, such as less than 10%, such as less than 5%, or less than 1 % of the initial rate determined using the equivalent DNA only oligonucleotide using the methodology provided by Example 91 - 95 of EP 1 222 309.

A compound is deemed capable of recruiting RNase H if, when provided with the complementary RNA target it has an initial rate, as measured in pmol/l/min, of at least 1% such as at least 5%, such as at least 10% or less than 20% of the equivalent DNA only oligonucleotide using the methodology provided by Example 91 - 95 of EP 1 222 309.

### miRNA and Disease

miRNAs that are associated with disease either through their upregulation or downregulation of transcripts may be used for diagnostic or therapeutic targets. For example, when expression of an miRNA results in downregulation or upregulation of a transcript associated with a disease, administration of an amount of a blocking nucleic acid of the invention sufficient to reduce the in vivo produces a therapeutic effect. Alternatively, a nucleic acid of the invention may be used in a diagnostic to determine whether the target is present in a sample from a subject, e.g., to determine risk for a disease caused by a miRNA binding to the target site or to determine suitability of a particular therapeutic. When underexpression of an miRNA or its target sequence is associated with a disease, nucleic acids of the invention may be used as diagnostics, as described herein. Exemplary diseases that are associated with miRNAs include cancer, heart disease, cardiovascular disease, neurological diseases such as Parkinson's disease, Alzheimer's, spinal muscular atrophy and X mental retardation, atherosclerosis, postangioplasty restenosis, transplantation arteriopathy, stroke, viral infection, psoriasis, metabolic disease, diabetes mellitus, and diabetic nephropathy. Specific miRNAs and diseases are further described below.

miRNAs are reported to be associated with the pathogenesis of a large range of human diseases (Soifer et al., Molecular Therapy 15(12); 2070-2079, 2007 for review). miRNA expression profiles demonstrate that many miRNAs are deregulated in human cancers. miRNAs have been shown to regulate oncogenes, tumor suppressors and a number of cancer-related genes controlling cell cycle, apoptosis, cell migration and angiogenesis. miRNAs encoded by the mir-17-92 cluster have oncogenic potential and others may act as tumor suppressors. Some miRNAs and their target sites have been found to be mutated in cancer (Calin and Croce, Nature Reviews 6; 857-866, 2006; Esquela-Kerscher and Slack, Nature Reviews 6; 259-269, 2006 for reviews)

Other specific miRNAs are misexpressed in diseased hearts, and gain- and loss-of-function experiments in mice have shown these miRNAs to be necessary and sufficient for multiple forms of heart disease (Rooij and Olsen, J Clin Invest 117: 2369-2376, 2007 for review).

Recent studies (Martin et al., JBC 282(33); 24262-24269, 2007) demonstrate that miR-155 translationally represses the expression of Angiotensin II type 1 receptor (AT1R) in vivo. A silent polymorphism in the human AT1R gene has been associated with cardiovascular disease.

MicroRNAs have recently been implicated in the intricate cross-talk between the host and pathogen in viral infections and is thought to play a major role in viral pathogenesis (Scaria et al., Retrovirology 3; 68, 2006 for review).

Recently, it has been shown that miRNA are active during embryogenesis of the mouse epithelium and play a significant role in skin morphogenesis (Yi et al., Nature Genetics 38(3); 273-274, 2006) and a strong indication for the involvement of miR-203 in the pathogenesis of psoriasis has been presented (Sonkoly et al., PLoS ONE. 2(7):e610, 2007).

The abundant expression of miRNAs in the brain highlights their biological significance in neurodevelopment. It was recently shown that miR-124 directly targets PTBP1 (PTB/hnRNP I) mRNA, which encodes a global repressor of alternative pre-mRNA splicing in nonneuronal cells and promotes the development of the nervous system (NS), at least in part by regulating an intricate network of NS-specific alternative splicing (Makeyev et al., Molecular Cell 27(3); 435-448, 2007-12-11).

Target prediction and in vitro functional studies have shown that MITF, a transcription factor required for the establishment and maintenance of retinal pigmented epithelium, is a direct target of miR-96 and miR-182 (Xu et al., JBC 282(34); 25053-25066, 2007).

Additionally microRNAs have been shown to play a role in insulin secretion and glucose homeostasis. Overexpression of miR-375 suppressed glucose-induced insulin secretion and inhibition of endogenous miR-375 function enhanced insulin secretion (Poy et al., Nature 432(7014); 226-30, 2004). MiR-375 was shown to target myotrophin (Mtpn) and inhibition of of Mtpn mimicked the effects of miR-375 on glucose-stimulated insulin secretion and exocytosis.

The liver-specific miR-122 was inhibited in mice with a 2'-O-methoxyethyl phosphorothioate antisense oligonucleotide resulting in reduced plasma cholesterol levels, increased hepatic fatty-acid oxidation, and a decrease in hepatic fatty-acid and cholesterol synthesis rates (Esau et al., Cell Me-tab. 3(2):87-98, 2006). miR-122 inhibition in a diet-induced obesity mouse model resulted in decreased plasma cholesterol levels and a significant improvement in liver steatosis, accompanied by reductions in several lipogenic genes. These results implicate miR-122 as a key regulator of cholesterol and fatty-acid metabolism in the adult liver and suggest that miR-122 may be an attractive therapeutic target for metabolic disease.

miR-21 is exemplary of a miRNA with well-characterized targets and a function associated with the progression of diseases, such as cancer.

miR-21 is strongly overexpressed in glioblastoma and mir-21 knockdown in cultured glioblastoma cells, triggers activation of caspases and leads to increased apoptotic cell death (Chan et al., Cancer Res 66: 6029-6033, 2005). miR-21 is also up-regulated in breast cancer (lorio et al., Cancer Res 65: 7065-7070, 2005) and cholangiocarcinomas (Meng et al., Gastroenterology 130: 2113-2129, 2006).

Previously, identified targets for miR-21 include the tumor suppressors Tropomysin 1 (TPM1) in breast cancer cells (Zhu et al., J Biol Chem 282(19); 14328-14336, 2007) and Phosphatase and tensin homolog (PTEN) in hepatocellular carcinomas (Meng et al., Gastroenterology 133(2); 647-658, 2007). Given the importance of PTEN in regulating the Phosphoinositide Kinase 3/AKT pathway and the frequency of PTEN mutations or silencing in a variety of cancers this constitutes an appealing explanation for the over-expression of miR-21 observed in many cancer types.

Recently, PDCD4, CDK6, and Cofilin2 were shown to be directly regulated by mir-21 (Frankel et al., J Biol Chem in press;1-10, 2007). PDCD4 is another tumor suppressor known to be upregulated during apoptosis and downregulated in several cancer forms such as lung cancer and hepatocellular carcinoma. We show herein that the miR-21 interaction with the miR-21 target site in the PDCD4 transcript can be inhibited by nucleic acids according to the present invention, providing specific inhibition of a miRNA target gene involved in disease.

An example of a miRNA that is frequently deregulated in cancer and target oncogenes is the let-7 miRNA family. lethal-7 (let-7) is temporally regulated in C. elegans, Drosophila, and zebrafish (Pasquinelli et al., Nature 408; 86-89, 2000; Reinhart et al., Nature 403; 901-906, 2000). In humans the let-7 family includes 12 homologues that have been found to map to regions deleted in human cancers (Calin et al., PNAS 101; 2999-3004, 2004) and let-7 is poorly expressed in lung cancers (Takamizawa et al., Cancer Res 64; 3753-3756, 2004).

All three human RAS oncogenes have let-7 complementary sites in their 3'UTR and the let-7 miRNA family was shown to directly regulate the RAS oncogene negatively (Johnson et al., Cell 120: 635-647, 2005) implicating a function for let-7 as a tumor suppressor in lung tissue.

Another target of let-7, HMGA2, a high-mobility group protein, is oncogenic in a variety of tumors, including benign mesenchymal tumors and lung cancers. HMGA2 was derepressed upon inhibition of let-7 in cells with high levels of the miRNA. Ectopic expression of let-7 reduced HMGA2 and cell proliferation in a lung cancer cell. The effect of let-7 on HMGA2 was dependent on multiple target sites in the 3' untranslated region (UTR), and the growth-suppressive effect of let-7 on lung cancer cells was rescued by overexpression of the HMGA2 ORF without a 3'UTR (Lee and Dutta, Genes & Development 21(9); 1025-1030, 2007).

In silico analysis of Disabled2 (Dab2), a putative tumor suppressor protein, 3' UTR revealed microRNA complimentary to this region of the gene, suggesting that microRNA mediated targeting of Dab2 mRNA might account for loss of the protein in breast cancer (Bagadi et al., Breast Cancer Research and Treatment 104(3); 277-286.

Estrogen receptor α (ERα) is a target of miR-206 in breast cancer cell lines (Adams et al., Molecular Endocrinology 21(5); 1132-1147, 2007).

Cases of chronic lymphocytic leukaemia (CLL) with good prognostic features typically are characterized down-regulation of genes miR-15a and miR-16-1, located at 13q14.3. Both microRNAs negatively regulate BCL2 at a post-transcriptional level. On the other hand, in CLL cases that use unmutated Ig heavy-chain variable-region genes (IgVH) or have high level expression of the 70-kD zeta-associated protein (ZAP-70) have high levels of TCL1 due to low-level expression of miR-29 and miR-181, which directly target this oncogene (Calin et al. Best Practice & Research, Clinical Hematology 20(3); 425-437, 2007).

Felli et al. (PNAS 102; 18081-18086, 2005) described the ability of miR-221 and miR-222 to downregulate the KIT oncogene to modulate erythropoiesis in CD34+ hematopoiteic progenitor cells and inhibit cell growth of TF1 erythroleukemic cell line. He et al. (PNAS 102; 19075-19080, 2005) found that patients with papillary thyroid carcinomas showed decreased KIT transcript and protein levels and reciprocally increased levels of miR-221, miR-222 and miR-146 in the tumours.

Galardi et al. (JBC 282(32); 23716-23724) present further indications that miR-221/222 can be regarded as a new family of oncogenes, directly targeting the tumor suppressor p27Kip1, and that their overexpression might be one of the factors contributing to the oncogenesis and progression of prostate carcinoma through p27Kip1 down-regulation.

mir-122a modulates cyclin G1 expression in hepatocellular carcinoma (HCC) derived cell lines and an inverse correlation between miR-122a and cyclin G1 expression exists in primary liver carcinomas (Gramantieri et al., Cancer Research 67(13); 6092-6099, 2007).

miRNAs are aberrantly expressed in the vascular cell walls after balloon injury (Ji et al., Circulation Research 100 (11); 1579-1588, 2007) and knock-down of miR-21 had a significant negative effeet on neointimal lesion formation. Western blot analysis demonstrated that PTEN and Bcl-2 were involved in miR-21 mediated cellular effects. The results suggest that miRNAs may be a new therapeutic target for proliferative vascular diseases such as atherosclerosis, postangioplasty restenosis, transplantation arteriopathy, and stroke.

Lecellier et al. (Science 308; 557-560, 2005) demonstrated that a mammalian microRNA, miR-32 restricts the accumulation of the retrovirus primate foamy virus type 1 (PFV-1) in human cells throwing light into the role of microRNAs in antiviral defense.

Computational methods incorporating both consensus prediction and target accessibility, have shown that human encoded microRNAs could target critical genes involved in the pathogenesis and tropism of influenza virus A/H5N1

WO2007042899 relates to the mapping of human microRNA targets in HIV genome including the nef gene which plays an important role in delayed disease progression (Hariharan et al., Bio-chem Biophys Res Commun 337(4):1214-8, 2005).

Stern-Ginossar et al. (Science 317(5836): 376-81, 2007) identified the major histocompatibility complex class I-related chain B (MICB) gene as a top candidate target of hcmv-miR-UL112, a human cytomegalovirus microRNA. MICB is a stress-induced ligand of the natural killer (NK) cell activating receptor NKG2D and is critical for the NK cell killing of virus-infected cells and tumor cells. It was shown that hcmv-miR-UL112 specifically down-regulates MICB expression during viral infection, leading to decreased binding of NKG2D and reduced killing by NK cells. The results reveal a miRNA-based immunoevasion mechanism that appears to be exploited by human cytomegalovirus.

Jopling et al. (Science 309; 1577-1581, 2005) reported a case wherein a liver specific microRNA miR-122 was shown to cause accumulation of viral RNA by binding to the 5' non-coding region of the viral genome of hepatitis C virus.

Both computational tools and experimental validation of the predicted candidates have shown that viruses also encode microRNAs. Viruses with miRNA mediated regulation include herpesvirus, HIV and Simian Virus 40. Bennasser et al. (Retrovirology 1;43, 2004) reported a computational screen for HIV-1 encoded microRNAs and further went about predicting their cellular targets and found five pre-miRNA candidates which has potential to encode 10 microRNAs and through them regulate -1000 host transcripts.

Of late, Cui et al. (J Virol 80; 5499-5508, 2006) discovered novel virus encoded microRNAs from HSV genome and Gupta et al. (Nature 442(7098):82-5, 2006) discovered that Herpes simplex-1 (HSV-1) latency associated transcript (LAT) encodes for a microRNA which target critical genes of the apoptosis pathway including TGF-β1 and SMAD3.

During the investigation of the function of miR-124 during spinal cord development, two endogenous targets of miR-124, laminγ1 and integrinβ1 were identified (Cao et al., Genes & Development 21(5); 531-536, 2007), both of which are highly expressed by neural progenitors but repressed upon neuronal differentation.

Kato et al. (PNAS 104(9); 3432-3437, 2007) uncovered a role for miRs in the kidney and diabetic nephropathy in controlling TGFβ-induced collagen1α1 and -2 expression by down-regulating E-box repressors. Smad-interacting protein 1 (SIP1), a E-box repressor, was shown to be a target of miR-192, a key miR highly expressed in the kidney, in mouse mesangial cells.

HMGA2 expression has been shown to be associated with enhanced selective chemosensitivity towards the topoisomerase II inhibitor, doxorubicin in cancer cells. Herbert et al (Molecular Cancer, 6, 2007) report that HMGA2 expression in head and neck squamous cell carcinoma cells is regulated in part by miR-98. Transfection of pre-miR-98 during normoxia diminishes HMGA2 and potentiates resistance to doxorubicin and cisplatin.

ZFHX1B is a transcriptional repressor involved in the TGFbeta signaling pathway and in processes of epithelial to mesenchymal transition via regulation of E-cadherin. It was shown that Zfhx1b and miR-200b are regionally coexpressed in the adult mouse brain and that miR-200b represses the expression of Zfhx1b via multiple sequence elements present in the 3'-untranslated region. Overexpression of miR-200b leads to repression of endogenous ZFHX1B, and inhibition of miR-200b relieves the repression of ZFHX1B (Christoffersen et al., RNA 13(8); 1172-1178, 2007).

Co-expression of the miR-17-92 cluster acted with c-myc expression to accelerate tumour development in a mouse B-cell lymphoma model (He et al., Nature 435; 828-833, 2005).

The E2F family of transcription factors is essential in the regulation of the cell cycle and apoptosis. E2F1 appears to be negatively regulated by miR-17-5p and miR-20a, two members of the mir-17-92 cluster (Lewis et al, Cell 115(7); 787-798, 2003).

miR-20a also modulates the translation of the E2F2 and E2F3 mRNAs via binding sites in their 3'UTR (Sylvestre et al., JBC 282(4); 2135-2143, 2007). Overexpression of miR-20a decreased apoptosis in a prostate cancer cell line pointing toward an anti-apoptotic role for miR-20a. In addition evidence suggesting an autoregulatory feedback loop between E2F factors and miRNAs from the mir-17-92 cluster has been presented.

The mir-17-92 cluster is overexpressed in lung cancers, especially in the most aggressive small-cell lung cancer (Hayashita et al., Cancer Res. 65; 9628-9632, 2005).

Ozen et al. (Oncogene, Sep 24, 2007, Epub ahead of print) found and verified widespread, but not universal, downregulation of miRNAs in clinically localized prostate cancer relative to benign peripheral zone tissue. The downregulated miRNAs include several with proven target mRNAs whose proteins have been previously shown to be increased in prostate cancer by immunohistochemistry, including RAS, E2F3, BCL-2 and MCL-1. Using a bioinformatics approach, they identified additional potential mRNA targets of one of the miRNAs, (miR-125b) that are upregulated in prostate cancer and confirmed increased expression of one of these targets, EIF4EBP1, in prostate cancer tissues.

The heart responds to diverse forms of stress by hypertrophic growth accompanied by fibrosis and eventual diminution of contractility, which results from down-regulation of α-myosin heavy chain (αMHC) and up-regulation of βMHC, the primary contractile proteins of the heart.

The cardiac-specific microRNA, miR-208, encoded by an intron of the αMHC gene was found to be required for cardiomyocyte hypertrophy, fibrosis and expression of βMHC in response to stress and hypothyroidism in miR-208 mutant mice (Rooji et al., Science 316; 575-579, 2007). Experiments indicated that a predicted miR-208 target, thyroid hormone receptor associated protein 1 (THRAP1), is negatively regulated by miR-208 implicating that miR-208 acts, at least in part, by repressing expression of the thyroid hormone receptor coregulator THRAP1, which can exert positive and negative effects on transcription.

Welch et al. (Oncogene 26(34): 5017-5022, 2007) has shown that miR-34a is generally expressed at lower levels in unfavorable primary neuroblastoma (NB) tumors and cell lines relative to normal adrenal tissue and that reintroduction of this miRNA into three different NB cell lines causes a dramatic reduction in cell proliferation through the induction of a caspase-dependent apoptotic pathway. As a potential mechanistic explanation for this observation, they also demonstrated that miR-34a directly targets the mRNA encoding E2F3 and significantly reduces the levels of E2F3 protein, a potent transcriptional inducer of cell-cycle progression. Furthermore, miR-34a expression increases during retinoic acid-induced differentiation of the SK-N-BE cell line, whereas E2F3 protein levels decrease. Thus, adding to the increasing role of miRNAs in cancer, miR-34a may act as a suppressor of NB tumorgenesis.

Using a miRNA microarrays platform and quantitative real time-polymerase chain reaction, miR-15a, miR-15b, miR-16-1, let-7a-3, let-7c, let-7d, miR-223, miR-342 and miR-107 was found to be upregulated in acute promyelocytic leukaemia patients and cell lines during all-trans-retinoic acid (ATRA) treatment, whereas miR-181b was downregulated (Garzon et al., Oncogene 26(28): 4148-4157, 2007). miR-107 was verified to target NFI-A, a gene that has been involved in a regulatory loop involving miR-223 and C/EBPa during granulocytic differentiation and ATRA downregulation of RAS and Bcl2 correlated with the activation of known miRNA regulators of those proteins, let-7a and miR-15a/miR-16-1, respectively.

Deregulation of the TCL1 oncogene is a causal event in the pathogenesis of the aggressive form B-cell chronic lymphocytic leukemia (B-CLL). Pekarsky et al. (Cancer Res. 66(24); 11590-11593, 2006) demonstrated that Tcl1 expression is regulated by miR-29 and miR-181, two microRNAs differentially expressed in CLL. Expression levels of miR-29 and miR-181 generally inversely correlated with Tcl1 expression in the examined CLL samples.

miRNA also target Cytochrome P450 (CYP), a superfamily of drug-metabolizing enzymes. Human CYP1B1, which is highly expressed in estrogen target tissues, catalyzes the metabolic activation of various procarcinogens and the 4-hydroxylation of 17beta-estradiol and was shown to be post-transcriptionally regulated by miR-27b (Tsuchiya et al., Cancer Res. 66(18); 9090-9098, 2006). In most breast cancer patients, the expression level of miR-27b was decreased in cancerous tissues, accompanied by a high level of CYP1B1 protein. A significant inverse association was observed between the expression levels of miR-27b and CYP1B1 protein.

Hossain et al. (Mol. Cell Biol. 26(21); 8191-201, 2006) reported a role for miR-17-5p as a tumor suppressor in breast cancer cells.

miR-17-5p has extensive complementarity to the mRNA of AIB1 (amplified in breast cancer 1). Cell culture experiments showed that AIB1 expression was downregulated by miR-17-5p, primarily through translational inhibition and that downregulation of AIB1 by Mir-17-5p resulted in decreased estrogen receptor-mediated, as well as estrogen receptor-independent, gene expression and decreased proliferation of breast cancer cells. miR-17-5p also completely abrogated the insulin-like growth factor 1-mediated, anchorage-independent growth of breast cancer cells.

In non-cell-autonomous Myc-induced tumor phenotypes, microRNAs have been shown to have a role involving the regulation of anti-angiogenic thrombospondin-1 (Tsp1) and connective tissue growth factor (CTGF), which are targets for repression by the miR-17-92 cluster, which is upregulated in colonic epithelial cells coexpressing K-Ras and c-Myc (Dews et al., Nature Genetics 38(9); 1060-1065, 2006).

Some patients who suffer from Tourettes' syndrome, a neuropsychiatric disorder characterized by persistent vocal and motor tics, have mutations in the miR-189 target site within the 3' UTR of the gene encoding SLITRK1, a single pass transmembrane protein with a leucine-rich extracellular domain (Abelson et al., Science 310, 317-320, 2005), providing an example that mutations in the 3' UTR of the candidate disease genes that disrupt specific miRNA binding sites can impact diseases through reduced or total loss of miRNA-mediated regulation.

### Pharmaceutical Compositions

The oligos of the invention may be formulated into pharmaceutical compositions for administration to human subjects in a biologically compatible form suitable for administration *in vivo.*

The oligos of the invention may be used in the form of the free acid, free base, in the form of salts, solvates, and as prodrugs. All forms are within the scope of the invention. In accordance with the methods of the invention, the described oligos or salts, solvates, or prodrugs thereof may be administered to a patient in a variety of forms depending on the selected route of administration, as will be understood by those skilled in the art. The oligos of the invention may be administered, for example, by oral, parenteral, buccal, sublingual, nasal, rectal, patch, pump, or transdermal administration and the pharmaceutical compositions formulated accordingly. Parenteral administration includes intravenous, intraperitoneal, subcutaneous, intramuscular, transepithelial, nasal, intrapulmonary, intrathecal, rectal, and topical modes of administration. Parenteral administration may be by continuous infusion over a selected period of time.

An oligo of the invention may be orally administered, for example, with an inert diluent or with an assimilable edible carrier, or it may be enclosed in hard or soft shell gelatin capsules, or it may be compressed into tablets, or it may be incorporated directly with the food of the diet. For oral therapeutic administration, an oligo of the invention may be incorporated with an excipient and used in the form of ingestible tablets, buccal tablets, troches, capsules, elixirs, suspensions, syrups, wafers, and the like.

An oligo of the invention may also be administered parenterally. Solutions of an oligo of the invention can be prepared in water suitably mixed with a surfactant, such as hydroxypropylcellulose. Dispersions can also be prepared in glycerol, liquid polyethylene glycols, DMSO and mixtures thereof with or without alcohol, and in oils. Under ordinary conditions of storage and use, these preparations may contain a preservative to prevent the growth of microorganisms. Conventional procedures and ingredients for the selection and preparation of suitable formulations are described, for example, in Remington's Pharmaceutical Sciences (2003 - 20th edition) and in The United States Pharmacopeia: The National Formulary (USP 24 NF19), published in 1999.

The pharmaceutical forms suitable for injectable use include sterile aqueous solutions or dispersions and sterile powders for the extemporaneous preparation of sterile injectable solutions or dispersions. In all cases the form must be sterile and must be fluid to the extent that may be easily administered via syringe.

Compositions for nasal administration may conveniently be formulated as aerosols, drops, gels, and powders. Aerosol formulations typically include a solution or fine suspension of the active substance in a physiologically acceptable aqueous or non-aqueous solvent and are usually presented in single or multidose quantities in sterile form in a sealed container, which can take the form of a cartridge or refill for use with an atomizing device. Alternatively, the sealed container may be a unitary dispensing device, such as a single dose nasal inhaler or an aerosol dispenser fitted with a metering valve which is intended for disposal after use. Where the dosage form comprises an aerosol dispenser, it will contain a propellant, which can be a compressed gas, such as compressed air or an organic propellant, such as fluorochlorohydrocarbon. The aerosol dosage forms can also take the form of a pump-atomizer.

Compositions suitable for buccal or sublingual administration include tablets, lozenges, and pastilles, where the active ingredient is formulated with a carrier, such as sugar, acacia, tragacanth, or gelatin and glycerine. Compositions for rectal administration are conveniently in the form of suppositories containing a conventional suppository base, such as cocoa butter.

The oligos of the invention may be administered to an animal alone or in combination with pharmaceutically acceptable carriers, as noted above, the proportion of which is determined by the solubility and chemical nature of the compound, chosen route of administration, and standard pharmaceutical practice.

The dosage of the oligos of the invention, and/or compositions comprising an oligo of the invention, can vary depending on many factors, such as the pharmacodynamic properties of the oligo; the mode of administration; the age, health, and weight of the recipient; the nature and extent of the symptoms; the frequency of the treatment, and the type of concurrent treatment, if any; and the clearance rate of the oligo in the animal to be treated. One of skill in the art can determine the appropriate dosage based on the above factors. The oligos of the invention may be administered initially in a suitable dosage that may be adjusted as required, depending on the clinical response.

In addition to the above-mentioned therapeutic uses, an oligo of the invention can also be used in diagnostic assays, screening assays, and as a research tool.

In diagnostic assays, an oligo of the invention may be useful in identifying or detecting a particular miRNA target sequence. For such a use, the oligo may be labelled, e.g., fluorescently labelled or radiolabelled, and contacted with a population of cells of an organism or a nucleic acid sample from an organism.

As another example, an isolated sample from a patient could be cultured ex vivo, and a blocking nucleic acid of the invention may be administered to the sample to modulate the interaction between a specific nucleic acid target (such as a mRNA) and a microRNA. In one application, a proposed treatment could be co-administered to test if modulating a specific microRNA would render the disease more or less sensitive to such proposed treatment.

The present oligonucleotides of the invention are furthermore useful and applicable for large-scale and genome-wide expression profiling of nucleotide targets to determine the prevalence of specific miRNA target site containing nucleotides by oligonucleotide microarrays.

In screening assays, an oligo of the invention may be used to identify other compounds that prevent an miRNA from binding to a particular target site. As research tools, the oligos of the invention may be used in enzyme assays and assays to study the localization of miRNA activity. Such information may be useful, for example, for diagnosing or monitoring disease states or progression. In such assays, an oligo of the invention may also be labelled.

### EXAMPLES

The invention will now be further illustrated with reference to the following examples. It will be appreciated that what follows is by way of example only and that modifications to detail may be made while still falling within the scope of the invention.

### Example 1. Synthesis, deprotection and purification of LNA-substituted oligonucleotides

LNA-substituted oligos were prepared on an automated DNA synthesizer (Expedite 8909 DNA synthesizer, PerSeptive Biosystems, 0.2 µmol scale) using the phosphoramidite approach (Beau-cage and Caruthers, Tetrahedron Lett. 22: 1859-1862, 1981) with 2-cyanoethyl protected LNA and DNA phosphoramidites, (Sinha, et al., Tetrahedron Lett.24: 5843-5846, 1983). CPG solid supports derivatised with a suitable quencher and 5'-fluorescein phosphoramidite (GLEN Research, Sterling, Virginia, USA). The synthesis cycle was modified for LNA phosphoramidites (250s coupling time) compared to DNA phosphoramidites. 1*H*-tetrazole or 4,5-dicyanoimidazole (Proligo, Hamburg, Germany) was used as activator in the coupling step.

The probes were deprotected using 32% aqueous ammonia (1h at room temperature, then 2 hours at 60°C) and purified by HPLC (Shimadzu-SpectraChrom series; Xterra™ RP18 column, 10?m 7.8 x 150 mm (Waters). Buffers: A: 0.05M Triethylammonium acetate pH 7.4. B. 50% acetonitrile in water. Eluent: 0-25 min: 10-80% B; 25-30 min: 80% B). The composition and purity of the probes were verified by MALDI-MS (PerSeptive Biosystem, Voyager DE-PRO) analysis.

### Example 2. Design of blocking molecules

Previous experiments using antagonizing oligos have demonstrated that important parameters for successful design are probe Tm and oligo self-annealling. If oligonucleotide Tm is too low, the efficiency is generally poor, maybe due to the oligo being removed from the target sequence by endogenous helicases. If Tm is too high, there is an increased risk that the oligo will anneal to partly complementary sites possibly leading to unspecific effects, known as off-targets effects. With respect to selfannealing (autocomplementarity) of the probe, a low selfannealing score (reflecting stability of the autoduplex) is favorable. Previous results have shown that probes exceeding a selfannealing score of about 45 often show very low potency or are completely nonfunctional. The effect of a high selfannealing score is a stable autoduplex which obviously sequestrates large amounts of probes, preventing the probe from interacting with its target sequence. To avoid high stability of the autoduplex, it is important to prevent LNA nucleotides in stretches of autocomplementary sequences. This may be acheived by an iterative approach in which the starting point is an oligonucleotide sequence consisting of only LNA monomers. This oligonculeotide is then put through a selfannealing scoring program (http://lnatools.com/hybridization/) that also identifies nucleotides participating in duplex formation. Next, one or more of these nucleotides are substituted with DNA, and the process is repeated, again substituting LNAs participating in duplex formation with DNA thereby gradually reducing selfannealing score. When reaching an appropriate Tm and selfannealing score, the process is stopped. The process is repeated for sequences spanning various regions of the target sequence to find optimal selfannealing scores and Tms.

An additional requirement in an oligonucleotide design process was the preference of LNA nucleotides in the terminals of the oligonucleotide. This was done to preserve biostability of the oligo, thereby improving duration of the biological response.

For the current experiments, three different oligos were designed to target the miR-21 target site in the PDCD4 transcript. These oligos vary slightly in target region and Tm (Figure 2). For each of the oligos a similar control oligo with approximately the same Tm and self-annealing score were also designed (see Table 1). Control oligos were designed to be fully complementary to a randomly chosen sequence approximately 600 bases downstream the predicted miRNA target site (Figure 2). The control oligos were designed to have comparable physicochemical properties as the miRNA targeting oligos.

**Table 1. Probe sequence and predicted Tm and selfannealing. The LNA monomers are uppercase letters.**

| **Oligonucleotide name** | **Oligo** | **Tm** | **Self annealing** |
|---|---|---|---|
| AHL_PDCD4-1 | AAAgGtaGcttaTtAGAAa | 66.9 | 37.0 |
| AHL_PDCD4-2 | CAAaAGgtaGcttaTtAGAAt | 72.0 | 45.0 |
| AHL_PDCD4-3 | AAgGtaGcttattAGAa | 61.2 | 31.0 |
| AHL_Control PDCD4-1 | ACAatggAgtaaAgTg | 62.6 | 35.0 |
| AHL_Control PDCD4-2 | ACAatgGagtaAagTGa | 66.0 | 33.0 |
| AHL_Control PDCD4-3 | ACAatgGagtaAaGTGAt | 69.6 | 29.0 |

### Example 3. Western blotting on cells transfected with blocking molecules.

MCF-7 (a human breast adenocarcinoma cell line) cells were maintained in Dulbecco's modified Eagle's medium (DMEM) with 10% FBS (Biochrom), 100 U/ml penicillin and 100 µg/ml streptomycin (Invitrogen) and incubated at 37°C in 5% CO₂. The cells were seeded in 6-well plates at a density of 300,000 cells/well. The following day, the cells were transfected with 50 nM of the indicated LNA constructs (PDCD4 1a, AHL_PDCD4-1; PDCD4 2, AHL_PDCD4-2; Control 2, AHL_Control PDCD4-2; Control 3, AHL_Control PDCD4-3; LNA SCR; LNA scramble (Exiqon, NB-1 negative, lot nr. EQ29093, 5'-TggTcaActGacAtaAcgTctmC-3', wherein capital letters denote LNA moieties and mC denotes LNA methyl cytosine)). The cell confluence upon the first transfection was approximately 50%. Transfections were performed in DMEM with 10% FBS without penicillin and streptomycin, using lipofectamine 2000 (Invitrogen). The amount of lipofectamine used per well was 2.5 µl. The cells were re-transfected a second time two days after the first transfection using the same procedure (cell confluence approximately 90%). Four days after the first transfection, cells were washed once in PBS and lysed in RIPA buffer (150mM NaCl, 1% NP40, 0.5% sodium deoxycholate, 0.1% SDS, 50mM Tris-HCI pH 8, 2mM EDTA) containing 1 mM DTT, 1 mM Pefabloc (Roche) and 1x Complete Mini protease inhibitor cocktail (Roche). 15 µg protein/lane was separated on a 12% polyacrylamide gel (200V for 1 hr) and transferred to a nitrocellulose membrane (200 mAMP for 1,5 hr). The membranes were blocked in 5% milk for 2 hrs at room temperature and subsequently incubated with primary antibodies overnight at 4°C. The PDCD4 antibody was diluted 1:400 and was kindly provided by Dr. Iwata Ozaki, Japan. The CDK6 antibody was diluted 1:200 and was purchased from Santa Cruz Biotechnology. The Vinculin antibody was diluted 1:150000 and was purchased from Sigma-Aldrich. The membranes were washed 3 x 10 min in PBS + 0.1% Tween and then incubated with secondary HRP-coupled antibodies (Vector Laboratories) (dilution 1:10000) for 1 hr at room temperature. The membranes were washed 3 x 10 min in PBS + 0.1% Tween and developed using the SuperSignal West Pico Chemiluminescent Substrate Kit (Pierce) (Figure 3). The bands were quantified relative to the appropriate Vinculin loading controls using a LAS-3000 imager (Fuji) and the relative quantifications are shown (LNA SCR=100).

MCF-7 cells have been shown to express high levels of miR-21 (Frankel et al., JBC Epub ahead of print, Nov 8 2007).

PDCD4 protein levels are up-regulated in MCF-7 cells transfected with blocking oligos partly complementary to the miR-21 target site in PDCD4 and partly complementary to non-target site sequences of the PDCD4 transcript as compared to the levels of PDCD4 protein in cells transfected with control oligos.

Protein levels of CDK6, which in addition to PDCD4 have been shown to be a target of miR-21 (Frankel et al., JBC Epub ahead of print, Nov 8 2007), are not significantly affected (Figure 3a) and in a parallel experiment, an up-regulation of both PDCD4 and CDK6 protein were observed when MCF-7 cells were transfected with a LNA oligonucleotide complementary to miR-21, LNA miR-21 (5'-tmCaamCatmCagTctGatAagmCta-3', wherein capital letters denote LNA moitites and mC denotes LNA methyl cytosine) (Figure 3b). These observations provide evidence that the blocking oligos specifically inhibit miR-21 interaction with the PDCD4 transcript.

### Example 4. Blocking of miR-21 target binding reporter assay

**Table 2.: Oligonucleotides and sequences used in the reporter assay:**

| **Oligonucleotide name** | **Oligonucleotide sequence** |
|---|---|
| Anti-21target Tm 73 | 5'-TAGmCTTATmCagAmCTGa-3' |
| Anti-21target Tm 70 | 5'-TAGmCTTATmCagAmCtGa-3' |
| Anti-21target Tm 75 | 5'-TAGmCTTatmCAgAmCtgATg-3' |
| Antitarget control Tm75 | 5'-AAmCTagTgmCgmCAgmCTTt-3' |
| Antitarget control Tm74 | 5'-AAmCTAgTgmCgmCAgmCt-3' |
| Antitarget control Tm71 | 5'-AAmCTagTgmCgmCAgmCt-3' |
| 2'OMe anti-21 target | **5'-tagcttatcagactgatg-3'** |
| 2'OMe control | **5'-aactagtgcgcagcttt-3'** |

a) Oligo nucleotide name and sequence. The LNA monomers are uppercase letters, mC is LNA methyl cytosine, DNA monomers are lowercase letters, and 2'OMe monomers are bold lower letters. For the LNA containing oligonucleotides the name indicates the predicted Tm according to LNA-DNA Tm prediction tool (Tolstrup et al., Nucleic Acids Res 31(13; 3758-3762, 2003).

LNA containing oligonucleotides were obtained from TIB MOLBIOL (www.Tibmolbiol.com), whereas 2'OMe was obtained from DNAtechnology (www.DNAtechnology.dk). All oligonucleotides were HPLC purified, and correct molecular mass was verified using mass spectroscopy.

### Design of oligonucleotides

LNA oligonucleotides were designed as described in Example 2. To investigate the effect of the predicted Tm and the inhibitory efficiency of the LNA containing oligonucleotides, three different oligonucleotides with various Tms were designed to be complementary to the miR-21 target site in the miR-21 reporter vector (pMIR-21) resulting in predicted Tms of 73 °C, 70°C, 75°C. Likewise three control oligonucleotides were designed and synthesized with similar predicted Tm (see table 2) but complementary to a region of the 3'UTR immediately adjacent to the miR-21 target site. This region is also present in the pMIR-16 control vector. The 2'OMe antitarget blocking and control oligonucleotides were designed to contain the same sequence as the LNA containing *Anti-21target Tm 75* and *Antitarget control Tm75* oligonucleotides as shown in table 2.

### b) miRNA reporter constructs

The pMIR-21 was constructed by inserting a miR-21 complementary sequence in the 3'UTR of the pMIR-REPORT (Ambion) containing the firefly luciferase reporter gene. This was done by annealing oligonucleotide I (A: 5'-AAT GCA CTA GTT CAA CAT CAG TCT GAT AAG CTA GCT CAG CAA GCT TAA TGC- 3') and II (B: 5'-GCA TTA AGC TTG CTG AGC TAG CTT ATC AGA CTG ATG TTG AAC TAG TGC ATT-3'). This fragment and the pMIR-REPORT vector were then digested with Spel and Hindlll, and the fragment was subsequently cloned into the Spel and Hindlll sites of pMIR-REPORT vector using standard techniques, thereby generating pMIR-21. The pMIR-16 was constructed using the same procedure but with the following DNA oligonucleotides for the insert: I (A: 5'-AAT GCA CTA GTC GCC AAT ATT TAC GTG CTG CTA GCT CAG CAA GCT TAA TGC- 3') and II (B: 5'-GCA TTA AGC TTG CTG AGC TAG CAG CAC GTA AATA TGG CGA CTA GTG CAT T-3').

### c) Reporter assays

HeLa and MCF7 cells were propagated in Dulbecco's Modified Eagle's Minimal Essential Medium (DMEM) with GlutamaxTM (Invitrogen) and supplemented with 10% foetal bovine serum (FBS). On the day prior to transfection cells were seeded in 96-well plates (Corning) at a density of 7000 cells/well. Cells were transfected using Xtreme Gene siRNA (Roche), with 70 ng/well of pMIR-21 reporter and 30 ng/well of the pGL4.73 Renilla (Promega) reporter plasmid for normalisation. Where indicated transfection mix also contained oligonucleotides resulting in a final concentration of 10 nM, 20 nM and 50 nM.

After 3-4h, media with transfection mix was removed and cells were washed four times in PBS and supplemented with fresh media. Luciferase activities (Firefly and Renilla) were measured 24 h later using the Dual Glow Luciferase kit (Promega) on a BMG Optima luminometer.

For the MCF7 cells, experiments were carried out as above, however these cells were propagated in Roswell Park Memorial Institute medium (RPMI) 1640 with GlutamaxTM (Invitrogen) and supplemented with 10% FBS. Cells were seeded to 15000 cell/well on the day prior to transfection and left for 48 h before measuring luciferase activity.

After luminescence measurements relative light units (RLU) were corrected for background and firefly luminescence (FL) was normalised to Renilla luminescence (RL). Data presented in the diagram show normalised FL activity as a function of oligonucleotide concentration and cell line.

### Results

To measure the effect of microRNA blocking oligonucleotides, a luciferase based miR-21 sensor reporter was constructed. This reporter harbours a sequence fully complementary to hsa-miR-21. When the reporter mRNA is recognized by a miR-21 containing RISC complex, the luciferase encoding mRNA is cleaved and subsequently degraded. The luciferase expression level thereby reflects the endogenous level and activity of miR-21. Likewise an identical control vector harbouring a 22 nt miR-16 complementary sequence was also constructed (pMIR-16).

In this line of experiments the pMIR-21 plasmid and control plasmid pMIR-16 were cotrans-fected with the miR-Target site blocking oligonucleotides (table 2), and reporter activity was subsequently measured.

Reporter data show that when co-transfected with miR-21 reporter plasmid all LNA containing oligonucleotides complementary to the miR-21 target site resulted in increased reporter activity (Fig. 4). Relative to the control oligonucleotides, reporter activity increased as much a 10-fold with a strong dose response not reaching saturation at 50 nM oligonucleotide concentration. None of the control oligonucleotides showed any significant effect on reporter activity despite being complementary to an adjacent 3'UTR sequence. This effect was evident for all three pairs of target site blocking and control oligonucleotides and was apparent in both MCF7 and HeLa cells.

In a control experiment, the miR-21 target site blocking oligonucletides and controls were co-transfected with the pMIR-16 reporter carrying a miR-16 complementary sequence whose activity is affected by the miR-16 expression level in the cell lines. Thus, this reporter is not a target for the miR-21, and miR-21 target site blocking oligonucleotides; however the vector is complementary to the control oligonucleotides targeting a 3'UTR sequence adjacent to the miR-target site.

In these experiments (Figure 5), reporter activity is only slightly affected by cotransfection with the miR-21 complementary oligonucleotides and shows no dose response curve in either cell line, demonstrating that no nonspecific sequence effect is generated by the miR-21 target site blocking oligonucleotides.

To address the effect of LNA oligonucleotides relative to 2'OMe modified oligonucleotides, a similar experiment was carried out using 2'OMe modified oligonucleotides as miR-21 target site blockers. Both miR-21 targeting and control oligonucleotides were designed to target identical sequence as the LNA containing oligonucleotides (see table 2).

The reporter results (Figure 4) show that a significant effect of reporter activity was not observed in either HeLa or MCF7 cell lines, indicating that 2'OMe oligonucleotides were not ideally suitable for conditions required for blocking access of RISC-miRNA complex to the target transcripts. This may have to do with lower duplex stability of the 2'OMe and RNA compared to LNA RNA duplexes or to the lower biostability of 2'OMe modified oligonucleotides relative to LNA containing oligonucleotides (Grünweller et al., Nucleic Acids Res. 31(12); 3185-3193, 2003).

All together these experiments demonstrate sequence specific regulation of the reporter activity of the LNA containing oligonucleotides both on the level of oligonucleotide sequence and vector sequence. The effect is strong in both HeLa and MCF7 cell lines, both known to express high levels of miR-21 and miR-16 (Blower et al., Mol Cancer Ther 6(5); 1483-1491, 2007 and Meister et al., RNA 10(3); 544-550, 2004). This demonstrates the suitability of LNA modified oligonucleotides for a miRNA target site blocking approach.

### Example 5

Poy et al. (Nature 432; 226-230, 2004) confirmed that Myotrophin (Mtpn) mRNA is a physiological target of the evolutionary conserved and islet-specific miRNA miR-375. They showed that miR-375 has a role in insulin secretion and glucose homeostasis leading to the view that miR-375 has the potential to be a novel target for therapeutic intervention in diabetes mellitus.

To further illustrate one aspect of the present invention, examples of blocking oligos for a microRNA target site for murine miR-375 in the gene transcript target gene Mtpn are depicted in figure 1c.

### Example 6

Rooij et al. (Science 316; 575-579, 2007) verified the mRNA encoding thyroid hormone receptor associated protein 1 (THRAP1) as a physiological target for miR-208, a miRNA expressed specifically in the heart and trace levels in the lung.

miR-208-/- mice showed reduced cardiac hypertrophy in response to pressure overload and the experiments suggested that THRAP1 contributes to effects of miR-208 on cardiac growth and gene expression. Sequences of miR-208 found in humans and mice were identical.

To further illustrate one aspect of the present invention, examples of blcking oligos for a microRNA target site for murine miR-208 in the gene transcript target gene THRAP1 are depicted in figure 1d.

## Claims

1. A method for producing a microRNA target site blocking nucleic acid directed at a specific transcript, wherein the oligonucleotide sequence of the nucleic acid overlaps the 5' or 3' end of the microRNA target site, and at the same time partly overlaps the non-microRNA target sites, the method comprising the steps of:
1) selecting a known microRNA having at least one identified or putative microRNA target site,
2) obtaining the sequence of said microRNA and the sequence of one of said microRNA target sites,
3) selecting and obtaining the sequence of a transcript which comprises the microRNA target site selected in step 2,
4) designing a microRNA target site blocking nucleic acid which is at least 10 nucleotides long and comprises at least one LNA analogue and which is either
a) at least partially complementary to the microRNA target site and which comprises a consecutive sequence of nucleotides which are complementary to the 3' end of the microRNA target site selected in step 3 and a consecutive sequence of nucleotides which are complementary to the transcript sequence immediately adjacent and downstream of the 3' end of the microRNA target site, or
b) at least partially complementary to the microRNA target site and which comprises a consecutive sequence of nucleotides which are complementary to end 5' end of the microRNA target site selected in step 3 and a consecutive sequence of nucleotides which are complementary to the transcript sequence immediately adjacent and upstream of the 5' end of the microRNA target site
5) synthesizing the nucleic acid designed in step 4.

2. The method according to claim 1 wherein the nucleic acid designed in step 4) is from 10 to 30 nucleotides long.

3. The method according to claim 1 wherein the nucleic acid designed in step 4) is from 15 to 30 nucleotides long.

4. The method according to any of the preceding claims which further comprises verifying the presence of the microRNA target site by the steps of:
a) contacting a nucleic acid sample from a subject with one or more nucleic acids produced by the method of claim 1, and
b) determining an expression level of the transcript comprising said target site or its translation product, wherein a change in the expression level of the transcript comprising the target site or its translation product verifies the presence of the microRNA target site,
c) if the presence of the microRNA target site is verified, then synthesizing the nucleic acid as described in step 5.

5. The method according to any of the preceding claims, wherein said microRNA target site is **characterized by** being complementary to 3-8 nucleotides of the seed sequence of the microRNA.

6. The method according to any of the preceding claims, further comprising aligning the transcript sequence and the sequence the microRNA target site selected in step 3.

7. The nucleic acid obtained by the methods of any of the preceding claims, wherein said nucleic acid is selected from the group of nucleic acids having the sequence SEQ ID NOs.: 4, 5, 6, 7, 8, 9, 12, 13, 14, 17, 18, 19, 21, 22 and 23.

8. A pharmaceutical composition comprising one or more nucleic acids of claim 7 and a pharmaceutically acceptable excipient.

9. The composition according to any of claims 7 or 8 for use as a medicament.

## Patentansprüche

1. Verfahren zur Herstellung von MicroRNA-Zielstellenblockierenden Nucleinsäuren, die auf ein spezifisches Transkript ausgerichtet sind, wobei die Oligonucleotidsequenz der Nucleinsäure das 5'- oder 3'-Ende der MicroRNA-Zielstelle überlagert und gleichzeitig teilweise die nicht-MicroRNA-Zielstellen überlagert, wobei das Verfahren die folgenden Schritte umfasst:
1) Auswählen einer bekannten MicroRNA-Zielstelle, die mindestens eine ermittelte oder vermeintliche MicroRNA-Zielstelle aufweist,
2) Erhalten der Sequenz der MicroRNA und der Sequenz einer der MicroRNA-Zielstellen,
3) Auswählen und Erhalten der Sequenz eines Transkripts, das die in Schritt 2 ausgewählte MicroRNA-Zielstelle umfasst,
4) Ausgestalten einer MicroRNA-Zielstelle, die eine Nucleinsäure blockiert, die mindestens 10 Nucleotide lang ist und mindestes ein LNA-Analogon aufweist und die entweder
a) mindestens teilweise komplementär zu der MicroRNA-Zielstelle ist und die eine aufeinanderfolgende Sequenz von Nucleotiden, die komplementär zu dem 3'-Ende der in Schritt 3 ausgewählten MicroRNA-Zielstelle sind, und eine aufeinanderfolgende Sequenz von Nucleotiden umfasst, die komplementär zu der Transkriptsequenz sind, die unmittelbar neben und stromabwärts von dem 3'-Ende der MicroRNA-Zielstelle liegt, oder
b) mindestens teilweise komplementär zu der MicroRNA-Zielstelle ist und die eine aufeinanderfolgende Sequenz von Nucleotiden, die komplementär zu dem 5'-Ende der in Schritt 3 ausgewählten MicroRNA-Zielstelle sind, und eine aufeinanderfolgende Sequenz von Nucleotiden umfasst, die komplementär zu der Transkriptsequenz sind, die unmittelbar neben und stromaufwärts von dem 5'-Ende der MicroRNA-Zielstelle liegt
5) Synthetisieren der in Schritt 4 ausgestalteten Nucleinsäure.

2. Verfahren nach Anspruch 1, wobei die in Schritt 4 ausgestaltete Nucleinsäure zwischen 10 und 30 Nucleotide lang ist.

3. Verfahren nach Anspruch 1, wobei die in Schritt 4 ausgestaltete Nucleinsäure zwischen 15 und 30 Nucleotide lang ist.

4. Verfahren nach einem der vorhergehenden Ansprüche, das ferner das Prüfen des Vorhandenseins der MicroRNA-Zielstelle durch die folgenden Schritte umfasst:
a) Zusammenbringen einer Nucleinsäure aus einem Subjekt mit einer oder mehreren Nucleinsäuren, die nach dem Verfahren aus Schritt 1 hergestellt werden, und
b) Bestimmen einer Expressionsstufe des Transkripts, das die Zielstelle oder ihr Translationsprodukt umfasst, wobei eine Veränderung der Expressionsstufe des Transkripts, das die Zielstelle oder ihr Translationsprodukt umfasst, das Vorhandensein der MicroRNA-Zielstelle bestätigt,
c) falls das Vorhandensein der MicroRNA-Zielstelle bestätigt ist, wird anschließend die Nucleinsäure wie in Schritt 5 beschrieben synthetisiert.

5. Verfahren nach einem der vorhergehenden Ansprüche, wobei die MicroRNA-Zielstelle **dadurch gekennzeichnet ist, dass** sie komplementär zu 3-8 Nucleotiden der Keimsequenz der MicroRNA ist.

6. Verfahren nach einem der vorhergehenden Ansprüche, das ferner das Ausrichten der Transkriptsequenz und der von der MicroRNA-Zielstelle in Schritt 3 ausgewählten Sequenz umfasst.

7. Durch die Verfahren der vorhergehenden Ansprüche erhaltene Nucleinsäure, wobei die Nucleinsäure ausgewählt ist aus der Gruppe von Nucleinsäuren, die die folgenden SEQ ID Nrn. der Sequenz aufweisen: 4, 5, 6, 7, 8, 9, 12, 13, 14, 17, 18, 19, 21, 22 und 23.

8. Pharmazeutische Zusammensetzung, die eine oder mehrere Nucleinsäuren nach Anspruch 7 und einen pharmazeutisch unbedenklichen Träger umfasst.

9. Zusammensetzung nach einem der Ansprüche 7 oder 8 zur Verwendung als ein Medikament.

## Revendications

1. Procédé de production d'un acide nucléique de blocage de sites cibles de microARN dirigé vers une transcription spécifique, dans lequel la séquence oligonucléotidique de l'acide nucléique chevauche l'extrémité 5' ou 3' du site cible de microARN, et chevauche en même temps partiellement les sites cibles non-microARN, le procédé comprenant les étapes :
1) de sélection d'un microARN connu ayant au moins un site cible de microARN identifié ou putatif,
2) d'obtention de la séquence dudit microARN et de la séquence de l'un desdits sites cibles de microARN,
3) de sélection et d'obtention de la séquence d'une transcription qui comprend le site cible de microARN sélectionné dans l'étape 2,
4) de conception d'un acide nucléique de blocage de sites cibles de microARN qui est long d'au moins 10 nucléotides et comprend au moins un analogue de LNA, et qui est soit
a) au moins partiellement complémentaire du site cible de microARN et qui comprend une séquence consécutive de nucléotides qui sont complémentaires de l'extrémité 3' du site cible de microARN sélectionné dans l'étape 3 et une séquence consécutive de nucléotides qui sont complémentaires de la séquence de transcription immédiatement adjacente et en aval de l'extrémité 3' du site cible de microARN, ou
b) au moins partiellement complémentaire du site cible de microARN et qui comprend une séquence consécutive de nucléotides qui sont complémentaires de l'extrémité 5' du site cible de microARN sélectionné dans l'étape 3 et une séquence consécutive de nucléotides qui sont complémentaires de la séquence de transcription immédiatement adjacente et en aval de l'extrémité 5' du site cible de microARN
5) de synthèse de l'acide nucléique conçu à l'étape 4.

2. Procédé selon la revendication 1, dans lequel l'acide nucléique conçu à l'étape 4) est long de 10 à 30 nucléotides.

3. Procédé selon la revendication 1, dans lequel l'acide nucléique conçu à l'étape 4) est long de 15 à 30 nucléotides.

4. Procédé selon l'une quelconque des revendications précédentes qui comprend en outre la vérification de la présence du site cible de microARN par les étapes :
a) de mise en contact d'un échantillon d'acide nucléique provenant d'un sujet avec un ou plusieurs acides nucléiques produits par le procédé selon la revendication 1, et
b) de détermination d'un niveau d'expression de la transcription comprenant ledit site cible ou son produit de translation, dans lequel un changement dans le niveau d'expression de la transcription comprenant le site cible ou son produit de translation vérifie la présence du site cible de microARN,
c) si la présence du site cible de microARN est vérifiée, alors de synthèse de l'acide nucléique comme décrit dans l'étape 5.

5. Procédé selon l'une quelconque des revendications précédentes, dans lequel ledit site cible de microARN est caractérisé comme étant complémentaire de 3 à 8 nucléotides de la séquence d'amorçage du microARN.

6. Procédé selon l'une quelconque des revendications précédentes, comprenant en outre l'alignement de la séquence de transcription et de la séquence du site cible de micro-ARN sélectionné dans l'étape 3.

7. Acide nucléique obtenu par les procédés selon l'une quelconque des revendications précédentes, dans lequel ledit acide nucléique est choisi dans le groupe d'acides nucléiques ayant la séquence SED ID NO : 4, 5, 6, 7, 8, 9, 12, 13, 14, 17, 18, 19, 21, 22 et 23.

8. Composition pharmaceutique comprenant un ou plusieurs acides nucléiques selon la revendication 7 et un excipient pharmaceutiquement acceptable.

9. Composition selon l'une quelconque des revendications 7 ou 8 destinée à être utilisée comme un médicament.


### SEQUENCE LISTING

<110> Exiqon A/S
<120> microRNA target site blocking oligos and uses thereof.
<130> 88.20-WO
<140> PCT/DK2007/000565
   <141> 2007-12-21
<150> DK PA 2006 01695
   <151> 2006-12-21
<160> 39
<170> PatentIn version 3.5
<210> 1
   <211> 22
   <212> RNA
   <213> Homo sapiens
<400> 1
   ugagguagua gguuguauag uu 22
<210> 2
   <211> 24
   <212> RNA
   <213> Homo sapiens
<400> 2
   aauaauacuu gccuaccuac cuca 24
<210> 3
   <211> 85
   <212> RNA
   <213> Homo sapiens
<400> 3
   ccucucugcc ucaguuuccc caucuguaaa augggagaau aauacuugcc uaccuaccuc 60
   acaggggugu ugugaggauu cauuu 85
<210> 4
   <211> 18
   <212> DNA
   <213> Artificial
<220>
   <223> Synthetic sequence
<220>
   <221> misc_feature
   <222> (1)..(1)
   <223> LNA guanine
<220>
   <221> misc_feature
   <222> (4)..(4)
   <223> LNA adenine
<220>
   <221> misc_feature
   <222> (8)..(8)
   <223> LNA thymine
<220>
   <221> misc_feature
   <222> (12)..(12)
   <223> LNA thymine
<220>
   <221> misc_feature
   <222> (18)..(18)
   <223> LNA adenine
<400> 4
   gcaactatta ttctccca 18
<210> 5
   <211> 18
   <212> DNA
   <213> Artificial
<220>
   <223> Synthetic sequence
<220>
   <221> misc_feature
   <222> (1)..(1)
   <223> LNA adenine
<220>
   <221> misc_feature
   <222> (6)..(6)
   <223> LNA adenine
<220>
   <221> misc_feature
   <222> (11)..(11)
   <223> LNA thymine
<220>
   <221> misc_feature
   <222> (15)..(15)
   <223> LNA adenine
<220>
   <221> misc_feature
   <222> (18)..(18)
   <223> LNA thymine
<400> 5
   acaacacccc tgtgaggt 18
<210> 6
   <211> 18
   <212> DNA
   <213> Artificial
<220>
   <223> Synthetic sequence
<220>
   <221> misc_feature
   <222> (1)..(1)
   <223> LNA adenine
<220>
   <221> misc_feature
   <222> (5)..(5)
   <223> LNA thymine
<220>
   <221> misc_feature
   <222> (9)..(9)
   <223> LNA thymine
<220>
   <221> misc_feature
   <222> (15)..(15)
   <223> LNA adenine
<220>
   <221> misc_feature
   <222> (18)..(18)
   <223> LNA thymine
<400> 6
   actattattc tcccattt 18
<210> 7
   <211> 18
   <212> DNA
   <213> Artificial
<220>
   <223> Synthetic sequence
<220>
   <221> misc_feature
   <222> (1)..(1)
   <223> LNA adenine
<220>
   <221> misc_feature
   <222> (6)..(6)
   <223> LNA thymine
<220>
   <221> misc_feature
   <222> (12)..(12)
   <223> LNA adenine
<220>
   <221> misc_feature
   <222> (15)..(15)
   <223> LNA thymine
<220>
   <221> misc_feature
   <222> (18)..(18)
   <223> LNA cytosine
<400> 7
   attattctcc cattttac 18
<210> 8
   <211> 18
   <212> DNA
   <213> Artificial
<220>
   <223> Synthetic sequence
<220>
   <221> misc_feature
   <222> (1)..(1)
   <223> LNA cytosine
<220>
   <221> misc_feature
   <222> (4)..(4)
   <223> LNA adenine
<220>
   <221> misc_feature
   <222> (9)..(9)
   <223> LNA adenine
<220>
   <221> misc_feature
   <222> (14)..(14)
   <223> LNA thymine
<220>
   <221> misc_feature
   <222> (18)..(18)
   <223> LNA adenine
<400> 8
   ctcacaacac ccctgtga 18
<210> 9
   <211> 18
   <212> DNA
   <213> Artificial
<220>
   <223> Synthetic sequence
<220>
   <221> misc_feature
   <222> (1)..(1)
   <223> LNA adenine
<220>
   <221> misc_feature
   <222> (4)..(4)
   <223> LNA cytosine
<220>
   <221> misc_feature
   <222> (7)..(7)
   <223> LNA adenine
<220>
   <221> misc_feature
   <222> (12)..(12)
   <223> LNA adenine
<220>
   <221> misc_feature
   <222> (18)..(18)
   <223> LNA guanine
<400> 9
   atcctcacaa cacccctg 18
<210> 10
   <211> 77
   <212> DNA
   <213> Mus musculus
<400> 10
   gctaactaat actgtgttta agtttcgtgt tgcaagaaca aatggaataa acttgaattg 60
   tgctatagat aacatgt 77
<210> 11
   <211> 22
   <212> RNA
   <213> Mus musculus
<400> 11
   uuuguucguu cggcucgcgu ga 22
<210> 12
   <211> 18
   <212> DNA
   <213> Artificial
<220>
   <223> Synthetic sequence
<220>
   <221> misc_feature
   <222> (1)..(1)
   <223> LNA cytosine
<220>
   <221> misc_feature
   <222> (2)..(3)
   <223> LNA adenine
<220>
   <221> misc_feature
   <222> (6)..(6)
   <223> LNA cytosine
<220>
   <221> misc_feature
   <222> (8)..(9)
   <223> LNA adenine
<220>
   <221> misc_feature
   <222> (11)..(11)
   <223> LNA cytosine
<220>
   <221> misc_feature
   <222> (14)..(14)
   <223> LNA adenine
<220>
   <221> misc_feature
   <222> (17)..(17)
   <223> LNA cytosine
<220>
   <221> misc_feature
   <222> (18)..(18)
   <223> LNA adenine
<400> 12
   caacacgaaa cttaaaca 18
<210> 13
   <211> 20
   <212> DNA
   <213> Artificial
<220>
   <223> Synthetic sequence
<220>
   <221> misc_feature
   <222> (1)..(1)
   <223> LNA cytosine
<220>
   <221> misc_feature
   <222> (2)..(3)
   <223> LNA adenine
<220>
   <221> misc_feature
   <222> (6)..(7)
   <223> LNA thymine
<220>
   <221> misc_feature
   <222> (10)..(10)
   <223> LNA thymine
<220>
   <221> misc_feature
   <222> (11)..(11)
   <223> LNA cytosine
<220>
   <221> misc_feature
   <222> (15)..(15)
   <223> LNA thymine
<220>
   <221> misc_feature
   <222> (19)..(19)
   <223> LNA thymine
<220>
   <221> misc_feature
   <222> (20)..(20)
   <223> LNA cytosine
<400> 13
   caagtttatt ccatttgttc 20
<210> 14
   <211> 21
   <212> DNA
   <213> Artificial
<220>
   <223> Synthetic sequence
<220>
   <221> misc_feature
   <222> (1)..(2)
   <223> LNA thymine
<220>
   <221> misc_feature
   <222> (5)..(5)
   <223> LNA thymine
<220>
   <221> misc_feature
   <222> (6)..(6)
   <223> LNA cytosine
<220>
   <221> misc_feature
   <222> (10)..(10)
   <223> LNA thymine
<220>
   <221> misc_feature
   <222> (12)..(12)
   <223> LNA guanine
<220>
   <221> misc_feature
   <222> (15)..(15)
   <223> LNA cytosine
<220>
   <221> misc_feature
   <222> (16)..(16)
   <223> LNA thymine
<220>
   <221> misc_feature
   <222> (18)..(18)
   <223> LNA guanine
<220>
   <221> misc_feature
   <222> (20)..(21)
   <223> LNA adenine
<400> 14
   ttattccatt tgttcttgca a 21
<210> 15
   <211> 78
   <212> DNA
   <213> Mus musculus
<400> 15
   cttcttgctt taaagcaatt ggtctaaaat atatgtaatc gtcttaatta aaacgttgca 60
   gtagggttgc ttttagag 78
<210> 16
   <211> 22
   <212> RNA
   <213> Mus musculus
<400> 16
   auaagacgag caaaaagcuu gu 22
<210> 17
   <211> 19
   <212> DNA
   <213> Artificial
<220>
   <223> Synthetic sequence
<220>
   <221> misc_feature
   <222> (1)..(1)
   <223> LNA thymine
<220>
   <221> misc_feature
   <222> (2)..(2)
   <223> LNA adenine
<220>
   <221> misc_feature
   <222> (4)..(4)
   <223> LNA adenine
<220>
   <221> misc_feature
   <222> (5)..(5)
   <223> LNA thymine
<220>
   <221> misc_feature
   <222> (7)..(7)
   <223> LNA thymine
<220>
   <221> misc_feature
   <222> (10)..(10)
   <223> LNA guanine
<220>
   <221> misc_feature
   <222> (11)..(11)
   <223> LNA adenine
<220>
   <221> misc_feature
   <222> (13)..(13)
   <223> LNA guanine
<220>
   <221> misc_feature
   <222> (15)..(15)
   <223> LNA adenine
<220>
   <221> misc_feature
   <222> (18)..(18)
   <223> LNA guanine
<220>
   <221> misc_feature
   <222> (19)..(19)
   <223> LNA cytosine
<400> 17
   tatatattag aggaattgc 19
<210> 18
   <211> 18
   <212> DNA
   <213> Artificial
<220>
   <223> Synthetic sequence
<220>
   <221> misc_feature
   <222> (1)..(1)
   <223> LNA guanine
<220>
   <221> misc_feature
   <222> (2)..(3)
   <223> LNA thymine
<220>
   <221> misc_feature
   <222> (6)..(7)
   <223> LNA adenine
<220>
   <221> misc_feature
   <222> (9)..(9)
   <223> LNA thymine
<220>
   <221> misc_feature
   <222> (12)..(12)
   <223> LNA guanine
<220>
   <221> misc_feature
   <222> (13)..(13)
   <223> LNA adenine
<220>
   <221> misc_feature
   <222> (15)..(15)
   <223> LNA guanine
<220>
   <221> misc_feature
   <222> (17)..(18)
   <223> LNA thymine
<400> 18
   gttttaatta agacgatt 18
<210> 19
   <211> 20
   <212> DNA
   <213> Artificial
<220>
   <223> Synthetic sequence
<220>
   <221> misc_feature
   <222> (1)..(1)
   <223> LNA thymine
<220>
   <221> misc_feature
   <222> (2)..(2)
   <223> LNA adenine
<220>
   <221> misc_feature
   <222> (4)..(4)
   <223> LNA thymine
<220>
   <221> misc_feature
   <222> (7)..(7)
   <223> LNA adenine
<220>
   <221> misc_feature
   <222> (10)..(10)
   <223> LNA cytosine
<220>
   <221> misc_feature
   <222> (13)..(14)
   <223> LNA thymine
<220>
   <221> misc_feature
   <222> (16)..(16)
   <223> LNA cytosine
<220>
   <221> misc_feature
   <222> (19)..(19)
   <223> LNA adenine
<220>
   <221> misc_feature
   <222> (20)..(20)
   <223> LNA thymine
<400> 19
   taattaagac gattacatat 20
<210> 20
   <211> 58
   <212> RNA
   <213> Homo sapiens
<400> 20
   caccuucucc gauuauucga uggaaaacau ucacgguaca aauaauagau uaguaagg 58
<210> 21
   <211> 19
   <212> DNA
   <213> Artificial
<220>
   <223> Synthetic sequence
<220>
   <221> misc_feature
   <222> (1)..(3)
   <223> LNA adenine
<220>
   <221> misc_feature
   <222> (5)..(5)
   <223> LNA guanine
<220>
   <221> misc_feature
   <222> (8)..(8)
   <223> LNA guanine
<220>
   <221> misc_feature
   <222> (13)..(13)
   <223> LNA thymine
<220>
   <221> misc_feature
   <222> (15)..(15)
   <223> LNA adenine
<220>
   <221> misc_feature
   <222> (16)..(16)
   <223> LNA guanine
<220>
   <221> misc_feature
   <222> (17)..(18)
   <223> LNA adenine
<400> 21
   aaaggtagct tattagaaa 19
<210> 22
   <211> 21
   <212> DNA
   <213> Artificial
<220>
   <223> synthetic sequence
<220>
   <221> misc_feature
   <222> (1)..(1)
   <223> LNA cytosine
<220>
   <221> misc_feature
   <222> (2)..(3)
   <223> LNA adenine
<220>
   <221> misc_feature
   <222> (5)..(5)
   <223> LNA adenine
<220>
   <221> misc_feature
   <222> (6)..(6)
   <223> LNA guanine
<220>
   <221> misc_feature
   <222> (10)..(10)
   <223> LNA guanine
<220>
   <221> misc_feature
   <222> (15)..(15)
   <223> LNA thymine
<220>
   <221> misc_feature
   <222> (17)..(17)
   <223> LNA adenine
<220>
   <221> misc_feature
   <222> (18)..(18)
   <223> LNA guanine
<220>
   <221> misc_feature
   <222> (19)..(20)
   <223> LNA adenine
<400> 22
   caaaaggtag cttattagaa t 21
<210> 23
   <211> 17
   <212> DNA
   <213> Artificial
<220>
   <223> synthetic sequence
<220>
   <221> misc_feature
   <222> (1)..(2)
   <223> LNA adenine
<220>
   <221> misc_feature
   <222> (4)..(4)
   <223> LNA guanine
<220>
   <221> misc_feature
   <222> (7)..(7)
   <223> LNA guanine
<220>
   <221> misc_feature
   <222> (14)..(14)
   <223> LNA adenine
<220>
   <221> misc_feature
   <222> (15)..(15)
   <223> LNA guanine
<220>
   <221> misc_feature
   <222> (16)..(16)
   <223> LNA adenine
<400> 23
   aaggtagctt attagaa 17
<210> 24
   <211> 57
   <212> RNA
   <213> Homo sapiens
<400> 24
   ccuuuuuuua cguccaucgu gaaaugaggu aacaauagac uggaucucga auuaauu 57
<210> 25
   <211> 16
   <212> DNA
   <213> Artificial
<220>
   <223> Synthetic sequence
<220>
   <221> misc_feature
   <222> (1)..(1)
   <223> LNA adenine
<220>
   <221> misc_feature
   <222> (2)..(2)
   <223> LNA cytosine
<220>
   <221> misc_feature
   <222> (3)..(3)
   <223> LNA adenine
<220>
   <221> misc_feature
   <222> (8)..(8)
   <223> LNA adenine
<220>
   <221> misc_feature
   <222> (13)..(13)
   <223> LNA adenine
<220>
   <221> misc_feature
   <222> (15)..(15)
   <223> LNA thymine
<400> 25
   acaatggagt aaagtg 16
<210> 26
   <211> 17
   <212> DNA
   <213> Artificial
<220>
   <223> Synthetic sequence
<220>
   <221> misc_feature
   <222> (1)..(1)
   <223> LNA adenine
<220>
   <221> misc_feature
   <222> (2)..(2)
   <223> LNA guanine
<220>
   <221> misc_feature
   <222> (3)..(3)
   <223> LNA adenine
<220>
   <221> misc_feature
   <222> (7)..(7)
   <223> LNA guanine
<220>
   <221> misc_feature
   <222> (12)..(12)
   <223> LNA adenine
<220>
   <221> misc_feature
   <222> (15)..(15)
   <223> LNA thymine
<220>
   <221> misc_feature
   <222> (16)..(16)
   <223> LNA guanine
<400> 26
   acaatggagt aaagtga 17
<210> 27
   <211> 18
   <212> DNA
   <213> Artificial
<220>
   <223> Synthetic sequence
<220>
   <221> misc_feature
   <222> (1)..(1)
   <223> LNA adenine
<220>
   <221> misc_feature
   <222> (2)..(2)
   <223> LNA guanine
<220>
   <221> misc_feature
   <222> (3)..(3)
   <223> LNA adenine
<220>
   <221> misc_feature
   <222> (7)..(7)
   <223> LNA guanine
<220>
   <221> misc_feature
   <222> (12)..(12)
   <223> LNA adenine
<220>
   <221> misc_feature
   <222> (14)..(14)
   <223> LNA guanine
<220>
   <221> misc_feature
   <222> (15)..(15)
   <223> LNA thymine
<220>
   <221> misc_feature
   <222> (16)..(16)
   <223> LNA guanine
<220>
   <221> misc_feature
   <222> (17)..(17)
   <223> LNA adenine
<400> 27
   acaatggagt aaagtgat 18
<210> 28
   <211> 16
   <212> DNA
   <213> Artificial
<220>
   <223> Synthetic sequence
<220>
   <221> misc_feature
   <222> (1)..(1)
   <223> LNA thymine
<220>
   <221> misc_feature
   <222> (2)..(2)
   <223> LNA adenine
<220>
   <221> misc_feature
   <222> (3)..(3)
   <223> LNA guanine
<220>
   <221> misc_feature
   <222> (4)..(4)
   <223> LNA 5-methyl cytosine
<220>
   <221> misc_feature
   <222> (5)..(6)
   <223> LNA thymine
<220>
   <221> misc_feature
   <222> (7)..(7)
   <223> LNA adenine
<220>
   <221> misc_feature
   <222> (8)..(8)
   <223> LNA thymine
<220>
   <221> misc_feature
   <222> (9)..(9)
   <223> LNA 5-methyl cytosine
<220>
   <221> misc_feature
   <222> (12)..(12)
   <223> LNA adenine
<220>
   <221> misc_feature
   <222> (13)..(13)
   <223> LNA 5-methyl cytosine
<220>
   <221> misc_feature
   <222> (14)..(14)
   <223> LNA thymine
<220>
   <221> misc_feature
   <222> (15)..(15)
   <223> LNA guanine
<400> 28
   tagcttatca gactga 16
<210> 29
   <211> 16
   <212> DNA
   <213> Artificial
<220>
   <223> Synthetic sequence
<220>
   <221> misc_feature
   <222> (1)..(1)
   <223> LNA thymine
<220>
   <221> misc_feature
   <222> (2)..(2)
   <223> LNA adenine
<220>
   <221> misc_feature
   <222> (3)..(3)
   <223> LNA guanine
<220>
   <221> misc_feature
   <222> (4)..(4)
   <223> LNA 5-methyl cytosine
<220>
   <221> misc_feature
   <222> (5)..(6)
   <223> LNA thymine
<220>
   <221> misc_feature
   <222> (7)..(7)
   <223> LNA adenine
<220>
   <221> misc_feature
   <222> (8)..(8)
   <223> LNA thymine
<220>
   <221> misc_feature
   <222> (9)..(9)
   <223> LNA 5-methyl cytosine
<220>
   <221> misc_feature
   <222> (12)..(12)
   <223> LNA adenine
<220>
   <221> misc_feature
   <222> (13)..(13)
   <223> LNA 5-methyl cytosine
<220>
   <221> misc_feature
   <222> (15)..(15)
   <223> LNA guanine
<400> 29
   tagcttatca gactga 16
<210> 30
   <211> 18
   <212> DNA
   <213> Artificial
<220>
   <223> Synthetic sequence
<220>
   <221> misc_feature
   <222> (1)..(1)
   <223> LNA thymine
<220>
   <221> misc_feature
   <222> (2)..(2)
   <223> LNA adenine
<220>
   <221> misc_feature
   <222> (3)..(3)
   <223> LNA guanine
<220>
   <221> misc_feature
   <222> (4)..(4)
   <223> LNA 5-methyl cytosine
<220>
   <221> misc_feature
   <222> (5)..(6)
   <223> LNA thymine
<220>
   <221> misc_feature
   <222> (9)..(9)
   <223> LNA 5-methyl cytosine
<220>
   <221> misc_feature
   <222> (10)..(10)
   <223> LNA adenine
<220>
   <221> misc_feature
   <222> (12)..(12)
   <223> LNA adenine
<220>
   <221> misc_feature
   <222> (13)..(13)
   <223> LNA 5-methyl cytosine
<220>
   <221> misc_feature
   <222> (16)..(16)
   <223> LNA adenine
<220>
   <221> misc_feature
   <222> (17)..(17)
   <223> LNA thymine
<400> 30
   tagcttatca gactgatg 18
<210> 31
   <211> 17
   <212> DNA
   <213> Artificial
<220>
   <223> synthetic sequence
<220>
   <221> misc_feature
   <222> (1)..(2)
   <223> LNA adenine
<220>
   <221> misc_feature
   <222> (3)..(3)
   <223> LNA 5-methyl cytosine
<220>
   <221> misc_feature
   <222> (4)..(4)
   <223> LNA thymine
<220>
   <221> misc_feature
   <222> (7)..(7)
   <223> LNA thymine
<220>
   <221> misc_feature
   <222> (9)..(9)
   <223> LNA 5-methyl cytosine
<220>
   <221> misc_feature
   <222> (11)..(11)
   <223> LNA 5-methyl cytosine
<220>
   <221> misc_feature
   <222> (12)..(12)
   <223> LNA adenine
<220>
   <221> misc_feature
   <222> (14)..(14)
   <223> LNA 5-methyl cytosine
<220>
   <221> misc_feature
   <222> (15)..(16)
   <223> LNA thymine
<400> 31
   aactagtgcg cagcttt 17
<210> 32
   <211> 15
   <212> DNA
   <213> Artificial
<220>
   <223> Synthetic sequence
<220>
   <221> misc_feature
   <222> (1)..(2)
   <223> LNA adenine
<220>
   <221> misc_feature
   <222> (3)..(3)
   <223> LNA 5-methyl cytosine
<220>
   <221> misc_feature
   <222> (4)..(4)
   <223> LNA thymine
<220>
   <221> misc_feature
   <222> (5)..(5)
   <223> LNA adenine
<220>
   <221> misc_feature
   <222> (7)..(7)
   <223> LNA thymine
<220>
   <221> misc_feature
   <222> (9)..(9)
   <223> LNA 5-methyl cytosine
<220>
   <221> misc_feature
   <222> (11)..(11)
   <223> LNA 5-methyl cytosine
<220>
   <221> misc_feature
   <222> (12)..(12)
   <223> LNA adenine
<220>
   <221> misc_feature
   <222> (14)..(14)
   <223> LNA 5-methyl cytosine
<400> 32
   aactagtgcg cagct 15
<210> 33
   <211> 15
   <212> DNA
   <213> Artificial
<220>
   <223> Synthetic sequence
<220>
   <221> misc_feature
   <222> (1)..(2)
   <223> LNA adenine
<220>
   <221> misc_feature
   <222> (3)..(3)
   <223> LNA 5-methyl cytosine
<220>
   <221> misc_feature
   <222> (4)..(4)
   <223> LNA thymine
<220>
   <221> misc_feature
   <222> (7)..(7)
   <223> LNA thymine
<220>
   <221> misc_feature
   <222> (9)..(9)
   <223> LNA 5-methyl cytosine
<220>
   <221> misc_feature
   <222> (11)..(11)
   <223> LNA 5-methyl cytosine
<220>
   <221> misc_feature
   <222> (12)..(12)
   <223> LNA adenine
<220>
   <221> misc_feature
   <222> (14)..(14)
   <223> LNA 5-methyl cytosine
<400> 33
   aactagtgcg cagct 15
<210> 34
   <211> 18
   <212> DNA
   <213> Artificial
<220>
   <223> Synthetic sequence
<220>
   <221> misc_feature
   <222> (1)..(18)
   <223> 2'-O-Methyl
<400> 34
   tagcttatca gactgatg 18
<210> 35
   <211> 17
   <212> DNA
   <213> Artificial
<220>
   <223> Synthetic sequence
<220>
   <221> misc_feature
   <222> (1)..(17)
   <223> 2'-O-Methyl
<400> 35
   aactagtgcg cagcttt 17
<210> 36
   <211> 51
   <212> DNA
   <213> Artificial
<220>
   <223> synthetic sequence
<400> 36
   aatgcactag ttcaacatca gtctgataag ctagctcagc aagcttaatg c 51
<210> 37
   <211> 51
   <212> DNA
   <213> Artificial
<220>
   <223> synthetic sequence
<400> 37
   gcattaagct tgctgagcta gcttatcaga ctgatgttga actagtgcat t 51
<210> 38
   <211> 51
   <212> DNA
   <213> Artificial
<220>
   <223> Synthetic sequence
<400> 38
   aatgcactag tcgccaatat ttacgtgctg ctagctcagc aagcttaatg c 51
<210> 39
   <211> 50
   <212> DNA
   <213> Artificial
<220>
   <223> synthetic sequence
<400> 39
   gcattaagct tgctgagcta gcagcacgta aatatggcga ctagtgcatt 50